# EUROPÄISCHE PATENTSCHRIFT

(11) **EP 1 670 920 B1**
(45) Veröffentlichungstag und Bekanntmachung des Hinweises auf die Patenterteilung: **05.03.2008**
(21) Anmeldenummer: 04765873.7
(22) Anmeldetag: 07.10.2004
(51) Int. Cl.: C12N 15/82

(54) **PROMOTOR ZUR EPIDERMISSPEZIFISCHEN TRANSGENEXPRESSION IN PFLANZEN**
PROMOTER FOR THE EPIDERMIS-SPECIFIC TRANSGENIC EXPRESSION IN PLANTS
PROMOTEUR FAVORISANT L'EXPRESSION TRANSGENIQUE SPECIFIQUE DE L'EPIDERME DES PLANTES

(30) Priorität: 07.10.2003 DE 10346611
(43) Veröffentlichungstag der Anmeldung: 21.06.2006
(73) Patentinhaber: Leibniz-Institut für Pflanzengenetik und Kulturpflanzenforschung (IPK), 06466 Gatersleben (DE); Max-Planck-Gesellschaft zur Förderung der Wissenschaften e.V., 80539 München (DE); Universität Zürich, 8006 Zürich (CH)
(72) Erfinder: SCHWEIZER, Patrick, 06493 Ballenstedt (DE); DUDLER, Robert, CH-8610 Uster (CH); SCHULZE-LEFERT, Paul, 50829 Köln (DE); PANSTRUGA, Ralph, 52066 Aachen (DE)
(74) Vertreter: Neuefeind, Regina
(86) Internationale Anmeldenummer: PCT/EP2004/011214
(87) Internationale Veröffentlichungsnummer: WO 2005/035766

(56) Entgegenhaltungen:
- MAUCH F. ET AL: "differential induction of distinct glutathione-S-transferase of wheat by xenobiotics and pathogen attack" PLANT PHYSIOL, Bd. 102, 1993, Seiten 1193-1201, XP002313139
- XU F. ET AL: "tandemly duplicated safener induced glutathione s-transferase genes from triticum tauschii contribute to genome and organe-specific expression in hexaploid wheat" PLANT PHYSIOL, Bd. 130, 2002, Seiten 362-373, XP002313140

### SEQUENZPROTOKOLL

<110> IPK Institut für Pflanzengenetik und Kulturpflanze
<120> Promotor zur epidermisspezifischen Transgenexpression in Pflanzen
<130> I 7469
<140> DE 103 46 611.8
   <141> 2003-10-07
<160> 15
<170> PatentIn Ver. 2.1
<210> 1
   <211> 2198
   <212> DNA
   <213> Triticum sp.
<400> 1
   gacgccgaag tggagccgac agcccccagg tcccaagccc tcggcagact agatcactag 60
   ccctggatcg gcgaggtgac tggatgacga gcagcacctg gtctggcggg tgttgggcga 120
   gtagaaccag gggcgatggc gacgcgctga ccttctcccc tcaccggcga tctgctcctt 180
   ctgggtgggg gtcgccggct gacgttctgt tgcggggtgg gggtcgccgg ctggcgttct 240
   gctgcggggt gggagtcgcc gaccggcgtg ctgctgctag gacaatcggt gaggccagtt 300
   aggtgctagc cgatcgattg gcgaagagat ccgagtcctg gggagatcag tgaggccagg 360
   tgctatttgg cctatcaatt ggccaggttc tgggaacggg gcgtggcgtg atcaacgagg 420
   tgctaggctg ctagctaggg aactggatcc tggaacgtgg aggaggcaag tccggtatgc 480
   taagtacttt aactttcctt cttcacatcc acctgattca gattattttg atctaaatta 540
   acttgcaaaa aatatatgtg tgatatccat ctactataat tgcttacaat caaaattata 600
   tgtgattttt tttagtttag aagatttata tgcacagtaa atctgaatgt tcttcacatg 660
   catgatttag tttaacttta aagagttata ctaactagtc ttgataaaga gatcttttgg 720
   agcaacacca aacctcgtga ggtgttttgc ctacggaaag gttgtgctat gtaatgatta 780
   ttattaggat caaagttgta ggataaacgt aaaaccttct cgatgtatct tttatacaac 840
   attgtagttt agttatatat ggagagagtg atttaacact ttgtgtttaa gagtagaata 900
   agttattcca cactctagcc aaacgaacta tttggcaaat atctcgctag ctggtgagag 960
   ccagagccgt ggaaagtctg tcttgctatt aaggcacaag catcaaacag gaacatttag 1020
   agccatggaa aagtgatgtg tcgcctacca atgggccaac tgctagcgat gtaataatag 1080
   catccaagtt gattttttat agaacatgca aggcgttggc aagtgggaaa atgattgatc 1140
   gctggcaagc ttaactctcg gaacttatag cattcaactg aatcagaaca aagattaaaa 1200
   aaaaatacat ttccatcgat agtgaaaaat tattcaattg agtgacaacg aaaatcatat 1260
   tggaatgtac atttacttgt tgattttaaa ttagaggcat ttttctacct tttttagtta 1320
   ataagatatg catataccca cccttagtgt tttcgagaca acgagagggc acattgcttt 1380
   tggtgctacc atctctctca agcctcaaat aagttgtgcg gacacgatta tcttcccgcg 1440
   ttggaatatc gtggcctggt agagctagcg aaaaatcttc catgttggaa tatgtcggca 1500
   gccggatagc cgccatgcat gtaaagtctc ttttaccttt acacttgctc aagtgacact 1560
   gtatgtcgcc taccacttgc taaatcaatg ggccaactgc tagcgacgta atagtagcaa 1620
   gttgatttac agtgttttgc tacagttctc tgactttgtt tcttcatttt agactagctg 1680
   actactgtcg cttacctgcc ttcccttctc cacgttagag gatccagttc tgatattgag 1740
   acctcgacga tgggaggaag ggcgcgatcg atgtggagta atttgaattt caaatctatc 1800
   tatctggggt atattggtcc ttcaccgatg tttggggggc tgtcggaaat tggttccgcg 1860
   atctacaaaa gtgaatggag ggagtagttg tttctccaat ccgtaccaac gcacgtgttt 1920
   ctaactagta cttacttcct tcgcaccaca atatggaata gagggagtat cgataaacta 1980
   acaaagatga ttacttaccc ggtttaaatg attcaagagc tcatttaatt tggcactcat 2040
   catttcatat atcttttttg gtagaaatga aataaagcag atctagacac tagctaaaaa 2100
   gtcgatgtag ccttgttatt tccttgggcc acgcgggccg ggtgtggtgc tccctgctct 2160
   gtgtataaat ggagatcaac atccaaggcc tcctccca                          2198
<210> 2
   <211> 114
   <212> DNA
   <213> Triticum sp.
<400> 2
   gtcagtcgtc ggacggtgtc cgttcatttc ctccccattt ttgtaattga ttaacttgtt 60
   atacatgctg acctcgacct gctgaataac gtccgtccat ggtttcccgt ccag       114
<210> 3
   <211> 2553
   <212> DNA
   <213> Triticum sp.
<400> 3
   gacgccgaag tggagccgac agcccccagg tcccaagccc tcggcagact agatcactag 60
   ccctggatcg gcgaggtgac tggatgacga gcagcacctg gtctggcggg tgttgggcga 120
   gtagaaccag gggcgatggc gacgcgctga ccttctcccc tcaccggcga tctgctcctt 180
   ctgggtgggg gtcgccggct gacgttctgt tgcggggtgg gggtcgccgg ctggcgttct 240
   gctgcggggt gggagtcgcc gaccggcgtg ctgctgctag gacaatcggt gaggccagtt 300
   aggtgctagc cgatcgattg gcgaagagat ccgagtcctg gggagatcag tgaggccagg 360
   tgctatttgg cctatcaatt ggccaggttc tgggaacggg gcgtggcgtg atcaacgagg 420
   tgctaggctg ctagctaggg aactggatcc tggaacgtgg aggaggcaag tccggtatgc 480
   taagtacttt aactttcctt cttcacatcc acctgattca gattattttg atctaaatta 540
   acttgcaaaa aatatatgtg tgatatccat ctactataat tgcttacaat caaaattata 600
   tgtgattttt tttagtttag aagatttata tgcacagtaa atctgaatgt tcttcacatg 660
   catgatttag tttaacttta aagagttata ctaactagtc ttgataaaga gatcttttgg 720
   agcaacacca aacctcgtga ggtgttttgc ctacggaaag gttgtgctat gtaatgatta 780
   ttattaggat caaagttgta ggataaacgt aaaaccttct cgatgtatct tttatacaac 840
   attgtagttt agttatatat ggagagagtg atttaacact ttgtgtttaa gagtagaata 900
   agttattcca cactctagcc aaacgaacta tttggcaaat atctcgctag ctggtgagag 960
   ccagagccgt ggaaagtctg tcttgctatt aaggcacaag catcaaacag gaacatttag 1020
   agccatggaa aagtgatgtg tcgcctacca atgggccaac tgctagcgat gtaataatag 1080
   catccaagtt gattttttat agaacatgca aggcgttggc aagtgggaaa atgattgatc 1140
   gctggcaagc ttaactctcg gaacttatag cattcaactg aatcagaaca aagattaaaa 1200
   aaaaatacat ttccatcgat agtgaaaaat tattcaattg agtgacaacg aaaatcatat 1260
   tggaatgtac atttacttgt tgattttaaa ttagaggcat ttttctacct tttttagtta 1320
   ataagatatg catataccca cccttagtgt tttcgagaca acgagagggc acattgcttt 1380
   tggtgctacc atctctctca agcctcaaat aagttgtgcg gacacgatta tcttcccgcg 1440
   ttggaatatc gtggcctggt agagctagcg aaaaatcttc catgttggaa tatgtcggca 1500
   gccggatagc cgccatgcat gtaaagtctc ttttaccttt acacttgctc aagtgacact 1560
   gtatgtcgcc taccacttgc taaatcaatg ggccaactgc tagcgacgta atagtagcaa 1620
   gttgatttac agtgttttgc tacagttctc tgactttgtt tcttcatttt agactagctg 1680
   actactgtcg cttacctgcc ttcccttctc cacgttagag gatccagttc tgatattgag 1740
   acctcgacga tgggaggaag ggcgcgatcg atgtggagta atttgaattt caaatctatc 1800
   tatctggggt atattggtcc ttcaccgatg tttggggggc tgtcggaaat tggttccgcg 1860
   atctacaaaa gtgaatggag ggagtagttg tttctccaat ccgtaccaac gcacgtgttt 1920
   ctaactagta cttacttcct tcgcaccaca atatggaata gagggagtat cgataaacta 1980
   acaaagatga ttacttaccc ggtttaaatg attcaagagc tcatttaatt tggcactcat 2040
   catttcatat atcttttttg gtagaaatga aataaagcag atctagacac tagctaaaaa 2100
   gtcgatgtag ccttgttatt tccttgggcc acgcgggccg ggtgtggtgc tccctgctct 2160
   gtgtataaat ggagatcaac atccaaggcc tcctcccaca cacacacgct acagagcaga 2220
   gcagagtctt gctccagtat ctgccctctc ctgcctgcct gtagagcatc catcacgtga 2280
   agttcacgga caaactacgt acacaggcag ctagctctcg aaacctcgct cgaaacgcac 2340
   ctgcagatcg ctctcttcgt cgtcgtcgcc gcgatcatca tcaacagctc cgtctgcctt 2400
   ggagccacgg ccgtccacga cgccgccgcc tcaggtcagt cgtcggacgg tgtccgttca 2460
   tttcctcccc atttttgtaa ttgattaact tgttatacat gctgacctcg acctgctgaa 2520
   taacgtccgt ccatggtttc ccgtccaggc acc                              2553
<210> 4
   <211> 1246
   <212> DNA
   <213> Triticum sp.
<400> 4
   accaccacac cactccacca gtaagaagtg cagcaggtag ctagtaagcc ggcgtagctt 60
   tgctcttgca gctagctagc taaccatggc cgcctctgcc tcttgccttt ctcttgtggt 120
   gctcgtggct ctggccacgg cggcgtcggc gcagctgtca ccgaccttct acgacacgtc 180
   ctgccccagg gccctggcca tcatcaagag tggcgtcatg gccgccgtga gcagcgaccc 240
   tcggatgggc gcgtcgctgc tccggctgca cttccacgac tgcttcgtcc aaggctgcga 300
   cgcgtctgtt ttgctgtctg gcatggaaca aaatgctatc ccgaacgcgg ggtcgctgag 360
   gggcttcggc gtcatcgaca gcatcaagac gcagatcgag gccatctgca atcagaccgt 420
   ctcctgcgcc gacatcctca ccgtcgccgc ccgtgactcc gttgtagccc tcggagggcc 480
   gtcatggaca gtccctctgg ggagaagaga ttccacagat gcaaacgagg cggcggcaaa 540
   cagcgacctg ccaggcttta catctagccg gtcagatctt gagctggcat tcagaaacaa 600
   gggcctcctt acgatcgaca tggtggccct ctcgggcgcg cacaccatcg gccaggcgca 660
   gtgtgggacc tttaaggaca ggatctacaa tgagactaac atcgacacgg ccttcgccac 720
   atctctccgg gccaactgcc ccaggtcaaa cggcgacggg agcctggcga acctggacac 780
   gacgacggcc aacacgttcg ataacgccta ctacaccaac ctcatgtcac agaaggggct 840
   cctgcactcg gaccaggtgc tgttcaacaa cgacaccacc gacaacactg tccggaactt 900
   tgcgtcgaac ccagcggcgt tcagcagcgc cttcacgacc gccatgatca agatgggcaa 960
   catcgcgccg aagacaggca cgcaggggca gatcaggctc agctgctcca gggtgaactc 1020
   gtgattgata gacgagttac tgcatactag ccagcacgac acgtacgtga atgaataagg 1080
   ccacagaacc agtggccaat ataaatacca gctcttgaaa ccgtgtattt tatgtacgag 1140
   tagcagcaaa tcatgcatgc atctacacat atatatgtaa cgatcgaatt cccactttct 1200
   catgcaaagg catggagaat tactatcaat cttagttata cgtgta                1246
<210> 5
   <211> 7011
   <212> DNA
   <213> Triticum sp.
<400> 5
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tgggtaccgg 660
   gccccccctc gagtctagaa ctagtggatc cccgacgccg aagtggagcc gacagccccc 720
   aggtcccaag ccctcggcag actagatcac tagccctgga tcggcgaggt gactggatga 780
   cgagcagcac ctggtctggc gggtgttggg cgagtagaac caggggcgat ggcgacgcgc 840
   tgaccttctc ccctcaccgg cgatctgctc cttctgggtg ggggtcgccg gctgacgttc 900
   tgttgcgggg tgggggtcgc cggctggcgt tctgctgcgg ggtgggagtc gccgaccggc 960
   gtgctgctgc taggacaatc ggtgaggcca gttaggtgct agccgatcga ttggcgaaga 1020
   gatccgagtc ctggggagat cagtgaggcc aggtgctatt tggcctatca attggccagg 1080
   ttctgggaac ggggcgtggc gtgatcaacg aggtgctagg ctgctagcta gggaactgga 1140
   tcctggaacg tggaggaggc aagtccggta tgctaagtac tttaactttc cttcttcaca 1200
   tccacctgat tcagattatt ttgatctaaa ttaacttgca aaaaatatat gtgtgatatc 1260
   catctactat aattgcttac aatcaaaatt atatgtgatt ttttttagtt tagaagattt 1320
   atatgcacag taaatctgaa tgttcttcac atgcatgatt tagtttaact ttaaagagtt 1380
   atactaacta gtcttgataa agagatcttt tggagcaaca ccaaacctcg tgaggtgttt 1440
   tgcctacgga aaggttgtgc tatgtaatga ttattattag gatcaaagtt gtaggataaa 1500
   cgtaaaacct tctcgatgta tcttttatac aacattgtag tttagttata tatggagaga 1560
   gtgatttaac actttgtgtt taagagtaga ataagttatt ccacactcta gccaaacgaa 1620
   ctatttggca aatatctcgc tagctggtga gagccagagc cgtggaaagt ctgtcttgct 1680
   attaaggcac aagcatcaaa caggaacatt tagagccatg gaaaagtgat gtgtcgccta 1740
   ccaatgggcc aactgctagc gatgtaataa tagcatccaa gttgattttt tatagaacat 1800
   gcaaggcgtt ggcaagtggg aaaatgattg atcgctggca agcttaactc tcggaactta 1860
   tagcattcaa ctgaatcaga acaaagatta aaaaaaaata catttccatc gatagtgaaa 1920
   aattattcaa ttgagtgaca acgaaaatca tattggaatg tacatttact tgttgatttt 1980
   aaattagagg catttttcta ccttttttag ttaataagat atgcatatac ccacccttag 2040
   tgttttcgag acaacgagag ggcacattgc ttttggtgct accatctctc tcaagcctca 2100
   aataagttgt gcggacacga ttatcttccc gcgttggaat atcgtggcct ggtagagcta 2160
   gcgaaaaatc ttccatgttg gaatatgtcg gcagccggat agccgccatg catgtaaagt 2220
   ctcttttacc tttacacttg ctcaagtgac actgtatgtc gcctaccact tgctaaatca 2280
   atgggccaac tgctagcgac gtaatagtag caagttgatt tacagtgttt tgctacagtt 2340
   ctctgacttt gtttcttcat tttagactag ctgactactg tcgcttacct gccttccctt 2400
   ctccacgtta gaggatccag ttctgatatt gagacctcga cgatgggagg aagggcgcga 2460
   tcgatgtgga gtaatttgaa tttcaaatct atctatctgg ggtatattgg tccttcaccg 2520
   atgtttgggg ggctgtcgga aattggttcc gcgatctaca aaagtgaatg gagggagtag 2580
   ttgtttctcc aatccgtacc aacgcacgtg tttctaacta gtacttactt ccttcgcacc 2640
   acaatatgga atagagggag tatcgataaa ctaacaaaga tgattactta cccggtttaa 2700
   atgattcaag agctcattta atttggcact catcatttca tatatctttt ttggtagaaa 2760
   tgaaataaag cagatctaga cactagctaa aaagtcgatg tagccttgtt atttccttgg 2820
   gccacgcggg ccgggtgtgg tgctccctgc tctgtgtata aatggagatc aacatccaag 2880
   gcctcctccc acacacacac gctacagagc agagcagagt cttgctccag tatctgccct 2940
   ctcctgcctg cctgtagagc atccatcacg tgaagttcac ggacaaacta cgtacacagg 3000
   cagctagctc tcgaaacctc gctcgaaacg cacctgcaga tcgctctctt cgtcgtcgtc 3060
   gccgcgatca tcatcaacag ctccgtctgc cttggagcca cggccgtcca cgacgccgcc 3120
   gcctcaggtc agtcgtcgga cggtgtccgt tcatttcctc cccatttttg taattgatta 3180
   acttgttata catgctgacc tcgacctgct gaataacgtc cgtccatggt ttcccgtcca 3240
   ggcaccccgg gctgcaggaa ttcaccacca caccactcca ccagtaagaa gtgcagcagg 3300
   tagctagtaa gccggcgtag ctttgctctt gcagctagct agctaaccat ggccgcctct 3360
   gcctcttgcc tttctcttgt ggtgctcgtg gctctggcca cggcggcgtc ggcgcagctg 3420
   tcaccgacct tctacgacac gtcctgcccc agggccctgg ccatcatcaa gagtggcgtc 3480
   atggccgccg tgagcagcga ccctcggatg ggcgcgtcgc tgctccggct gcacttccac 3540
   gactgcttcg tccaaggctg cgacgcgtct gttttgctgt ctggcatgga acaaaatgct 3600
   atcccgaacg cggggtcgct gaggggcttc ggcgtcatcg acagcatcaa gacgcagatc 3660
   gaggccatct gcaatcagac cgtctcctgc gccgacatcc tcaccgtcgc cgcccgtgac 3720
   tccgttgtag ccctcggagg gccgtcatgg acagtccctc tggggagaag agattccaca 3780
   gatgcaaacg aggcggcggc aaacagcgac ctgccaggct ttacatctag ccggtcagat 3840
   cttgagctgg cattcagaaa caagggcctc cttacgatcg acatggtggc cctctcgggc 3900
   gcgcacacca tcggccaggc gcagtgtggg acctttaagg acaggatcta caatgagact 3960
   aacatcgaca cggccttcgc cacatctctc cgggccaact gccccaggtc aaacggcgac 4020
   gggagcctgg cgaacctgga cacgacgacg gccaacacgt tcgataacgc ctactacacc 4080
   aacctcatgt cacagaaggg gctcctgcac tcggaccagg tgctgttcaa caacgacacc 4140
   accgacaaca ctgtccggaa ctttgcgtcg aacccagcgg cgttcagcag cgccttcacg 4200
   accgccatga tcaagatggg caacatcgcg ccgaagacag gcacgcaggg gcagatcagg 4260
   ctcagctgct ccagggtgaa ctcgtgattg atagacgagt tactgcatac tagccagcac 4320
   gacacgtacg tgaatgaata aggccacaga accagtggcc aatataaata ccagctcttg 4380
   aaaccgtgta ttttatgtac gagtagcagc aaatcatgca tgcatctaca catatatatg 4440
   taacgatcga attcccactt tctcatgcaa aggcatggag aattactatc aatcttagtt 4500
   atacgtgtat aaaaagcggc cgcgaattcg atatcaagct tatcgatacc gtcgacctcg 4560
   acctgcaggc atgcccgctg aaatcaccag tctctctcta caaatctatc tctctctata 4620
   ataatgtgtg agtagttccc agataaggga attagggttc ttatagggtt tcgctcatgt 4680
   gttgagcata taagaaaccc ttagtatgta tttgtatttg taaaatactt ctatcaataa 4740
   aatttctaat tcctaaaacc aaaatccagg ggtaccgagc tcgaattcta gtctacgcgg 4800
   ccgcgagctc cagcttttgt tccctttagt gagggttaat tgcgcgcttg gcgtaatcat 4860
   ggtcatagct gtttcctgtg tgaaattgtt atccgctcac aattccacac aacatacgag 4920
   ccggaagcat aaagtgtaaa gcctggggtg cctaatgagt gagctaactc acattaattg 4980
   cgttgcgctc actgcccgct ttccagtcgg gaaacctgtc gtgccagctg cattaatgaa 5040
   tcggccaacg cgcggggaga ggcggtttgc gtattgggcg ctcttccgct tcctcgctca 5100
   ctgactcgct gcgctcggtc gttcggctgc ggcgagcggt atcagctcac tcaaaggcgg 5160
   taatacggtt atccacagaa tcaggggata acgcaggaaa gaacatgtga gcaaaaggcc 5220
   agcaaaaggc caggaaccgt aaaaaggccg cgttgctggc gtttttccat aggctccgcc 5280
   cccctgacga gcatcacaaa aatcgacgct caagtcagag gtggcgaaac ccgacaggac 5340
   tataaagata ccaggcgttt ccccctggaa gctccctcgt gcgctctcct gttccgaccc 5400
   tgccgcttac cggatacctg tccgcctttc tcccttcggg aagcgtggcg ctttctcata 5460
   gctcacgctg taggtatctc agttcggtgt aggtcgttcg ctccaagctg ggctgtgtgc 5520
   acgaaccccc cgttcagccc gaccgctgcg ccttatccgg taactatcgt cttgagtcca 5580
   acccggtaag acacgactta tcgccactgg cagcagccac tggtaacagg attagcagag 5640
   cgaggtatgt aggcggtgct acagagttct tgaagtggtg gcctaactac ggctacacta 5700
   gaaggacagt atttggtatc tgcgctctgc tgaagccagt taccttcgga aaaagagttg 5760
   gtagctcttg atccggcaaa caaaccaccg ctggtagcgg tggttttttt gtttgcaagc 5820
   agcagattac gcgcagaaaa aaaggatctc aagaagatcc tttgatcttt tctacggggt 5880
   ctgacgctca gtggaacgaa aactcacgtt aagggatttt ggtcatgaga ttatcaaaaa 5940
   ggatcttcac ctagatcctt ttaaattaaa aatgaagttt taaatcaatc taaagtatat 6000
   atgagtaaac ttggtctgac agttaccaat gcttaatcag tgaggcacct atctcagcga 6060
   tctgtctatt tcgttcatcc atagttgcct gactccccgt cgtgtagata actacgatac 6120
   gggagggctt accatctggc cccagtgctg caatgatacc gcgagaccca cgctcaccgg 6180
   ctccagattt atcagcaata aaccagccag ccggaagggc cgagcgcaga agtggtcctg 6240
   caactttatc cgcctccatc cagtctatta attgttgccg ggaagctaga gtaagtagtt 6300
   cgccagttaa tagtttgcgc aacgttgttg ccattgctac aggcatcgtg gtgtcacgct 6360
   cgtcgtttgg tatggcttca ttcagctccg gttcccaacg atcaaggcga gttacatgat 6420
   cccccatgtt gtgcaaaaaa gcggttagct ccttcggtcc tccgatcgtt gtcagaagta 6480
   agttggccgc agtgttatca ctcatggtta tggcagcact gcataattct cttactgtca 6540
   tgccatccgt aagatgcttt tctgtgactg gtgagtactc aaccaagtca ttctgagaat 6600
   agtgtatgcg gcgaccgagt tgctcttgcc cggcgtcaat acgggataat accgcgccac 6660
   atagcagaac tttaaaagtg ctcatcattg gaaaacgttc ttcggggcga aaactctcaa 6720
   ggatcttacc gctgttgaga tccagttcga tgtaacccac tcgtgcaccc aactgatctt 6780
   cagcatcttt tactttcacc agcgtttctg ggtgagcaaa aacaggaagg caaaatgccg 6840
   caaaaaaggg aataagggcg acacggaaat gttgaatact catactcttc ctttttcaat 6900
   attattgaag catttatcag ggttattgtc tcatgagcgg atacatattt gaatgtattt 6960
   agaaaaataa acaaataggg gttccgcgca catttccccg aaaagtgcca c 7011
<210> 6
   <211> 746
   <212> DNA
   <213> Triticum sp.
<400> 6
   agcttattac atagcaagca tggggtactc caaaacccta gtagctggcc tgttcgcaat 60
   gctgttacta gctccggccg tcttggccac cgacccagac cctctccagg acttctgtgt 120
   cgccgacctc gacggcaagg cggtctcggt gaacgggcac acgtgcaagc ccatgtcgga 180
   ggccggcgac gacttcctct tctcgtccaa gttggccaag gccggcaaca cgtccacccc 240
   gaacggctcc gccgtgacgg agctcgacgt ggccgagtgg cccggtacca acacgctggg 300
   tgtgtccatg aaccgcgtgg actttgctcc cggaggcacc aacccaccac acatccaccc 360
   gcgtgccacc gagatcggca tcgtgatgaa aggtgagctt ctcgtgggaa tccttggcag 420
   cctcgactcc gggaacaagc tctactcgag ggtggtgcgc gccggagaga cgttcctcat 480
   cccacggggc ctcatgcact tccagttcaa cgtcggtaag accgaggcct ccatggtcgt 540
   ctccttcaac agccagaacc ccggcattgt cttcgtgccc ctcacgctct tcggctccaa 600
   cccgcccatc ccaacgccgg tgctcaccaa ggcactccgg gtggaggcca gggtcgtgga 660
   acttctcaag tccaagtttg ccgctgggtt ttaatttcta ggagccttcc ctgaaatgat 720
   aattatataa ttccatatat gcatgc 746
<210> 7
   <211> 6452
   <212> DNA
   <213> Triticum sp.
<400> 7
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tgggtaccgg 660
   gccccccctc gagtctagaa ctagtggatc cccgacgccg aagtggagcc gacagccccc 720
   aggtcccaag ccctcggcag actagatcac tagccctgga tcggcgaggt gactggatga 780
   cgagcagcac ctggtctggc gggtgttggg cgagtagaac caggggcgat ggcgacgcgc 840
   tgaccttctc ccctcaccgg cgatctgctc cttctgggtg ggggtcgccg gctgacgttc 900
   tgttgcgggg tgggggtcgc cggctggcgt tctgctgcgg ggtgggagtc gccgaccggc 960
   gtgctgctgc taggacaatc ggtgaggcca gttaggtgct agccgatcga ttggcgaaga 1020
   gatccgagtc ctggggagat cagtgaggcc aggtgctatt tggcctatca attggccagg 1080
   ttctgggaac ggggcgtggc gtgatcaacg aggtgctagg ctgctagcta gggaactgga 1140
   tcctggaacg tggaggaggc aagtccggta tgctaagtac tttaactttc cttcttcaca 1200
   tccacctgat tcagattatt ttgatctaaa ttaacttgca aaaaatatat gtgtgatatc 1260
   catctactat aattgcttac aatcaaaatt atatgtgatt ttttttagtt tagaagattt 1320
   atatgcacag taaatctgaa tgttcttcac atgcatgatt tagtttaact ttaaagagtt 1380
   atactaacta gtcttgataa agagatcttt tggagcaaca ccaaacctcg tgaggtgttt 1440
   tgcctacgga aaggttgtgc tatgtaatga ttattattag gatcaaagtt gtaggataaa 1500
   cgtaaaacct tctcgatgta tcttttatac aacattgtag tttagttata tatggagaga 1560
   gtgatttaac actttgtgtt taagagtaga ataagttatt ccacactcta gccaaacgaa 1620
   ctatttggca aatatctcgc tagctggtga gagccagagc cgtggaaagt ctgtcttgct 1680
   attaaggcac aagcatcaaa caggaacatt tagagccatg gaaaagtgat gtgtcgccta 1740
   ccaatgggcc aactgctagc gatgtaataa tagcatccaa gttgattttt tatagaacat 1800
   gcaaggcgtt ggcaagtggg aaaatgattg atcgctggca agcttaactc tcggaactta 1860
   tagcattcaa ctgaatcaga acaaagatta aaaaaaaata catttccatc gatagtgaaa 1920
   aattattcaa ttgagtgaca acgaaaatca tattggaatg tacatttact tgttgatttt 1980
   aaattagagg catttttcta ccttttttag ttaataagat atgcatatac ccacccttag 2040
   tgttttcgag acaacgagag ggcacattgc ttttggtgct accatctctc tcaagcctca 2100
   aataagttgt gcggacacga ttatcttccc gcgttggaat atcgtggcct ggtagagcta 2160
   gcgaaaaatc ttccatgttg gaatatgtcg gcagccggat agccgccatg catgtaaagt 2220
   ctcttttacc tttacacttg ctcaagtgac actgtatgtc gcctaccact tgctaaatca 2280
   atgggccaac tgctagcgac gtaatagtag caagttgatt tacagtgttt tgctacagtt 2340
   ctctgacttt gtttcttcat tttagactag ctgactactg tcgcttacct gccttccctt 2400
   ctccacgtta gaggatccag ttctgatatt gagacctcga cgatgggagg aagggcgcga 2460
   tcgatgtgga gtaatttgaa tttcaaatct atctatctgg ggtatattgg tccttcaccg 2520
   atgtttgggg ggctgtcgga aattggttcc gcgatctaca aaagtgaatg gagggagtag 2580
   ttgtttctcc aatccgtacc aacgcacgtg tttctaacta gtacttactt ccttcgcacc 2640
   acaatatgga atagagggag tatcgataaa ctaacaaaga tgattactta cccggtttaa 2700
   atgattcaag agctcattta atttggcact catcatttca tatatctttt ttggtagaaa 2760
   tgaaataaag cagatctaga cactagctaa aaagtcgatg tagccttgtt atttccttgg 2820
   gccacgcggg ccgggtgtgg tgctccctgc tctgtgtata aatggagatc aacatccaag 2880
   gcctcctccc acacacacac gctacagagc agagcagagt cttgctccag tatctgccct 2940
   ctcctgcctg cctgtagagc atccatcacg tgaagttcac ggacaaacta cgtacacagg 3000
   cagctagctc tcgaaacctc gctcgaaacg cacctgcaga tcgctctctt cgtcgtcgtc 3060
   gccgcgatca tcatcaacag ctccgtctgc cttggagcca cggccgtcca cgacgccgcc 3120
   gcctcaggtc agtcgtcgga cggtgtccgt tcatttcctc cccatttttg taattgatta 3180
   acttgttata catgctgacc tcgacctgct gaataacgtc cgtccatggt ttcccgtcca 3240
   ggcaccccgg gggatccagc ttattacata gcaagcatgg ggtactccaa aaccctagta 3300
   gctggcctgt tcgcaatgct gttactagct ccggccgtct tggccaccga cccagaccct 3360
   ctccaggact tctgtgtcgc cgacctcgac ggcaaggcgg tctcggtgaa cgggcacacg 3420
   tgcaagccca tgtcggaggc cggcgacgac ttcctcttct cgtccaagtt ggccaaggcc 3480
   ggcaacacgt ccaccccgaa cggctccgcc gtgacggagc tcgacgtggc cgagtggccc 3540
   ggtaccaaca cgctgggtgt gtccatgaac cgcgtggact ttgctcccgg aggcaccaac 3600
   ccaccacaca tccacccgcg tgccaccgag atcggcatcg tgatgaaagg tgagcttctc 3660
   gtgggaatcc ttggcagcct cgactccggg aacaagctct actcgagggt ggtgcgcgcc 3720
   ggagagacgt tcctcatccc acggggcctc atgcacttcc agttcaacgt cggtaagacc 3780
   gaggcctcca tggtcgtctc cttcaacagc cagaaccccg gcattgtctt cgtgcccctc 3840
   acgctcttcg gctccaaccc gcccatccca acgccggtgc tcaccaaggc actccgggtg 3900
   gaggccaggg tcgtggaact tctcaagtcc aagtttgccg ctgggtttta atttctagga 3960
   gccttccctg aaatgataat tatataattc catatatgca tgcctgcagg catgcccgct 4020
   gaaatcacca gtctctctct acaaatctat ctctctctat aataatgtgt gagtagttcc 4080
   cagataaggg aattagggtt cttatagggt ttcgctcatg tgttgagcat ataagaaacc 4140
   cttagtatgt atttgtattt gtaaaatact tctatcaata aaatttctaa ttcctaaaac 4200
   caaaatccag gggtaccgag ctcgaattct agtctacgcg gccgcgagct ccagcttttg 4260
   ttccctttag tgagggttaa ttgcgcgctt ggcgtaatca tggtcatagc tgtttcctgt 4320
   gtgaaattgt tatccgctca caattccaca caacatacga gccggaagca taaagtgtaa 4380
   agcctggggt gcctaatgag tgagctaact cacattaatt gcgttgcgct cactgcccgc 4440
   tttccagtcg ggaaacctgt cgtgccagct gcattaatga atcggccaac gcgcggggag 4500
   aggcggtttg cgtattgggc gctcttccgc ttcctcgctc actgactcgc tgcgctcggt 4560
   cgttcggctg cggcgagcgg tatcagctca ctcaaaggcg gtaatacggt tatccacaga 4620
   atcaggggat aacgcaggaa agaacatgtg agcaaaaggc cagcaaaagg ccaggaaccg 4680
   taaaaaggcc gcgttgctgg cgtttttcca taggctccgc ccccctgacg agcatcacaa 4740
   aaatcgacgc tcaagtcaga ggtggcgaaa cccgacagga ctataaagat accaggcgtt 4800
   tccccctgga agctccctcg tgcgctctcc tgttccgacc ctgccgctta ccggatacct 4860
   gtccgccttt ctcccttcgg gaagcgtggc gctttctcat agctcacgct gtaggtatct 4920
   cagttcggtg taggtcgttc gctccaagct gggctgtgtg cacgaacccc ccgttcagcc 4980
   cgaccgctgc gccttatccg gtaactatcg tcttgagtcc aacccggtaa gacacgactt 5040
   atcgccactg gcagcagcca ctggtaacag gattagcaga gcgaggtatg taggcggtgc 5100
   tacagagttc ttgaagtggt ggcctaacta cggctacact agaaggacag tatttggtat 5160
   ctgcgctctg ctgaagccag ttaccttcgg aaaaagagtt ggtagctctt gatccggcaa 5220
   acaaaccacc gctggtagcg gtggtttttt tgtttgcaag cagcagatta cgcgcagaaa 5280
   aaaaggatct caagaagatc ctttgatctt ttctacgggg tctgacgctc agtggaacga 5340
   aaactcacgt taagggattt tggtcatgag attatcaaaa aggatcttca cctagatcct 5400
   tttaaattaa aaatgaagtt ttaaatcaat ctaaagtata tatgagtaaa cttggtctga 5460
   cagttaccaa tgcttaatca gtgaggcacc tatctcagcg atctgtctat ttcgttcatc 5520
   catagttgcc tgactccccg tcgtgtagat aactacgata cgggagggct taccatctgg 5580
   ccccagtgct gcaatgatac cgcgagaccc acgctcaccg gctccagatt tatcagcaat 5640
   aaaccagcca gccggaaggg ccgagcgcag aagtggtcct gcaactttat ccgcctccat 5700
   ccagtctatt aattgttgcc gggaagctag agtaagtagt tcgccagtta atagtttgcg 5760
   caacgttgtt gccattgcta caggcatcgt ggtgtcacgc tcgtcgtttg gtatggcttc 5820
   attcagctcc ggttcccaac gatcaaggcg agttacatga tcccccatgt tgtgcaaaaa 5880
   agcggttagc tccttcggtc ctccgatcgt tgtcagaagt aagttggccg cagtgttatc 5940
   actcatggtt atggcagcac tgcataattc tcttactgtc atgccatccg taagatgctt 6000
   ttctgtgact ggtgagtact caaccaagtc attctgagaa tagtgtatgc ggcgaccgag 6060
   ttgctcttgc ccggcgtcaa tacgggataa taccgcgcca catagcagaa ctttaaaagt 6120
   gctcatcatt ggaaaacgtt cttcggggcg aaaactctca aggatcttac cgctgttgag 6180
   atccagttcg atgtaaccca ctcgtgcacc caactgatct tcagcatctt ttactttcac 6240
   cagcgtttct gggtgagcaa aaacaggaag gcaaaatgcc gcaaaaaagg gaataagggc 6300
   gacacggaaa tgttgaatac tcatactctt cctttttcaa tattattgaa gcatttatca 6360
   gggttattgt ctcatgagcg gatacatatt tgaatgtatt tagaaaaata aacaaatagg 6420
   ggttccgcgc acatttcccc gaaaagtgcc ac 6452
<210> 8
   <211> 1939
   <212> DNA
   <213> Triticum sp.
<400> 8
   ccactgtcca cacgaaatgt gccatctgaa acgcgttctg gaacagcgtc aggtgtatga 60
   agaagaggac ccagtcgggg cggtggaacc agaagaactt gttgctgggc tcgaccacgg 120
   gtgccccctt gatgacgctc gaccggtcct ggatctccag ggccatctcc atgatgatca 180
   tctctagctt ggttccaaca cacaagagga tgatgagagg gatgaaagaa acccaggtga 240
   gtgtgccgat cccgtcgata tcaaggaaga gggtgaggat cgccacagcc cacagcggga 300
   ggctgcgaaa agaggccaaa tgtgtcaaga tcatgcaaca aggaccagca ggggcaaaga 360
   ccatgacgca gcaaactgat agtattgtat catatggaag ctaagcaata tcatatggag 420
   cctgacgaca ctcgtgccga attcgattcg tgaatttcta gagaacaaaa ggtatgcatc 480
   aatttagaaa aaagtacact attatgtgat gtttgtttcc tatgctagtg gaacggatta 540
   gaattttttt ttcattaagg tcacctttac tggcataagc agttcacact aaacggtaaa 600
   ccttataggt gaaaattttc aggcatatat atatatatat atatatatat atgtttgatt 660
   ctttccggct taacaaaata attagcaagt acttcttgtt gcatttgttc caacggctga 720
   atttattggc atcggtccaa gaaatccatc taaatgtttt acatttcacc aaagtgtgtg 780
   tcatgacaga tgtaacaaat aataaaccaa aaggagagga aggaaagagg aagataaatg 840
   ttacaaaaat ttaaatcaaa cttatttcta cctttctcct tacctaccca gtttaaaaac 900
   acatattata ttttaaagag aggcaacatg cgccaaaggc tacccttgaa aattcctaaa 960
   atattgtaca tttgactgat gaccaaacaa aaagttaaat tgtctcttcc ttatcacatt 1020
   atatttccat gcatgccttt ttctggaaac ttactatcag caaaatttag atgaaaggat 1080
   aatgccacat aatttcagtc tccaagagat ttgttagttg tcatatatta aattggtggg 1140
   ccaatctatt cctgggtctt tttatgtatc tacttgacca tttgaacttc tgtagttaat 1200
   tgtattctat gaatgatcac tcatccaaaa acttgttatt tgtgttttac tctgttgaat 1260
   cttgaatatt tattcatttt gttcatcata cgattggagg cccataatag atgcttaatg 1320
   agagtaagat tatcgatctc caaacacatg cttcttacta gtgttgaata tatacccttt 1380
   tagatgtata gttcaaccca tagattcata tgaccctcag ctttctgatg tgtatgtatg 1440
   accttacact gacactctga actaatgtag gtatcttgtc ctgcaggaat tcggcacgag 1500
   tgtcgtcagg ctccatatga tattgcttag cttccatatg atacaatact atcagtttgc 1560
   tgcgtcatgg tctttgcccc tgctggtcct tgttgcatga tcttgacaca tttggcctct 1620
   tttcgcagcc tcccgctgtg ggctgtggcg atcctcaccc tcttccttga tatcgacggg 1680
   atcggcacac tcacctgggt ttctttcatc cctctcatca tcctcttgtg tgttggaacc 1740
   aagctagaga tgatcatcat ggagatggcc ctggagatcc aggaccggtc gagcgtcatc 1800
   aagggggcac ccgtggtcga gcccagcaac aagttcttct ggttccaccg ccccgactgg 1860
   gtcctcttct tcatacacct gacgctgttc cagaacgcgt ttcagatggc acatttcgtg 1920
   tggacaggca tgcgactgg 1939
<210> 9
   <211> 7633
   <212> DNA
   <213> Triticum sp.
<400> 9
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tgggtaccgg 660
   gccccccctc gagtctagaa ctagtggatc cccgacgccg aagtggagcc gacagccccc 720
   aggtcccaag ccctcggcag actagatcac tagccctgga tcggcgaggt gactggatga 780
   cgagcagcac ctggtctggc gggtgttggg cgagtagaac caggggcgat ggcgacgcgc 840
   tgaccttctc ccctcaccgg cgatctgctc cttctgggtg ggggtcgccg gctgacgttc 900
   tgttgcgggg tgggggtcgc cggctggcgt tctgctgcgg ggtgggagtc gccgaccggc 960
   gtgctgctgc taggacaatc ggtgaggcca gttaggtgct agccgatcga ttggcgaaga 1020
   gatccgagtc ctggggagat cagtgaggcc aggtgctatt tggcctatca attggccagg 1080
   ttctgggaac ggggcgtggc gtgatcaacg aggtgctagg ctgctagcta gggaactgga 1140
   tcctggaacg tggaggaggc aagtccggta tgctaagtac tttaactttc cttcttcaca 1200
   tccacctgat tcagattatt ttgatctaaa ttaacttgca aaaaatatat gtgtgatatc 1260
   catctactat aattgcttac aatcaaaatt atatgtgatt ttttttagtt tagaagattt 1320
   atatgcacag taaatctgaa tgttcttcac atgcatgatt tagtttaact ttaaagagtt 1380
   atactaacta gtcttgataa agagatcttt tggagcaaca ccaaacctcg tgaggtgttt 1440
   tgcctacgga aaggttgtgc tatgtaatga ttattattag gatcaaagtt gtaggataaa 1500
   cgtaaaacct tctcgatgta tcttttatac aacattgtag tttagttata tatggagaga 1560
   gtgatttaac actttgtgtt taagagtaga ataagttatt ccacactcta gccaaacgaa 1620
   ctatttggca aatatctcgc tagctggtga gagccagagc cgtggaaagt ctgtcttgct 1680
   attaaggcac aagcatcaaa caggaacatt tagagccatg gaaaagtgat gtgtcgccta 1740
   ccaatgggcc aactgctagc gatgtaataa tagcatccaa gttgattttt tatagaacat 1800
   gcaaggcgtt ggcaagtggg aaaatgattg atcgctggca agcttaactc tcggaactta 1860
   tagcattcaa ctgaatcaga acaaagatta aaaaaaaata catttccatc gatagtgaaa 1920
   aattattcaa ttgagtgaca acgaaaatca tattggaatg tacatttact tgttgatttt 1980
   aaattagagg catttttcta ccttttttag ttaataagat atgcatatac ccacccttag 2040
   tgttttcgag acaacgagag ggcacattgc ttttggtgct accatctctc tcaagcctca 2100
   aataagttgt gcggacacga ttatcttccc gcgttggaat atcgtggcct ggtagagcta 2160
   gcgaaaaatc ttccatgttg gaatatgtcg gcagccggat agccgccatg catgtaaagt 2220
   ctcttttacc tttacacttg ctcaagtgac actgtatgtc gcctaccact tgctaaatca 2280
   atgggccaac tgctagcgac gtaatagtag caagttgatt tacagtgttt tgctacagtt 2340
   ctctgacttt gtttcttcat tttagactag ctgactactg tcgcttacct gccttccctt 2400
   ctccacgtta gaggatccag ttctgatatt gagacctcga cgatgggagg aagggcgcga 2460
   tcgatgtgga gtaatttgaa tttcaaatct atctatctgg ggtatattgg tccttcaccg 2520
   atgtttgggg ggctgtcgga aattggttcc gcgatctaca aaagtgaatg gagggagtag 2580
   ttgtttctcc aatccgtacc aacgcacgtg tttctaacta gtacttactt ccttcgcacc 2640
   acaatatgga atagagggag tatcgataaa ctaacaaaga tgattactta cccggtttaa 2700
   atgattcaag agctcattta atttggcact catcatttca tatatctttt ttggtagaaa 2760
   tgaaataaag cagatctaga cactagctaa aaagtcgatg tagccttgtt atttccttgg 2820
   gccacgcggg ccgggtgtgg tgctccctgc tctgtgtata aatggagatc aacatccaag 2880
   gcctcctccc acacacacac gctacagagc agagcagagt cttgctccag tatctgccct 2940
   ctcctgcctg cctgtagagc atccatcacg tgaagttcac ggacaaacta cgtacacagg 3000
   cagctagctc tcgaaacctc gctcgaaacg cacctgcaga tcgctctctt cgtcgtcgtc 3060
   gccgcgatca tcatcaacag ctccgtctgc cttggagcca cggccgtcca cgacgccgcc 3120
   gcctcaggtc agtcgtcgga cggtgtccgt tcatttcctc cccatttttg taattgatta 3180
   acttgttata catgctgacc tcgacctgct gaataacgtc cgtccatggt ttcccgtcca 3240
   ggcaccccgg gccactgtcc acacgaaatg tgccatctga aacgcgttct ggaacagcgt 3300
   caggtgtatg aagaagagga cccagtcggg gcggtggaac cagaagaact tgttgctggg 3360
   ctcgaccacg ggtgccccct tgatgacgct cgaccggtcc tggatctcca gggccatctc 3420
   catgatgatc atctctagct tggttccaac acacaagagg atgatgagag ggatgaaaga 3480
   aacccaggtg agtgtgccga tcccgtcgat atcaaggaag agggtgagga tcgccacagc 3540
   ccacagcggg aggctgcgaa aagaggccaa atgtgtcaag atcatgcaac aaggaccagc 3600
   aggggcaaag accatgacgc agcaaactga tagtattgta tcatatggaa gctaagcaat 3660
   atcatatgga gcctgacgac actcgtgccg aattcgattc gtgaatttct agagaacaaa 3720
   aggtatgcat caatttagaa aaaagtacac tattatgtga tgtttgtttc ctatgctagt 3780
   ggaacggatt agaatttttt tttcattaag gtcaccttta ctggcataag cagttcacac 3840
   taaacggtaa accttatagg tgaaaatttt caggcatata tatatatata tatatatata 3900
   tatgtttgat tctttccggc ttaacaaaat aattagcaag tacttcttgt tgcatttgtt 3960
   ccaacggctg aatttattgg catcggtcca agaaatccat ctaaatgttt tacatttcac 4020
   caaagtgtgt gtcatgacag atgtaacaaa taataaacca aaaggagagg aaggaaagag 4080
   gaagataaat gttacaaaaa tttaaatcaa acttatttct acctttctcc ttacctaccc 4140
   agtttaaaaa cacatattat attttaaaga gaggcaacat gcgccaaagg ctacccttga 4200
   aaattcctaa aatattgtac atttgactga tgaccaaaca aaaagttaaa ttgtctcttc 4260
   cttatcacat tatatttcca tgcatgcctt tttctggaaa cttactatca gcaaaattta 4320
   gatgaaagga taatgccaca taatttcagt ctccaagaga tttgttagtt gtcatatatt 4380
   aaattggtgg gccaatctat tcctgggtct ttttatgtat ctacttgacc atttgaactt 4440
   ctgtagttaa ttgtattcta tgaatgatca ctcatccaaa aacttgttat ttgtgtttta 4500
   ctctgttgaa tcttgaatat ttattcattt tgttcatcat acgattggag gcccataata 4560
   gatgcttaat gagagtaaga ttatcgatct ccaaacacat gcttcttact agtgttgaat 4620
   atataccctt ttagatgtat agttcaaccc atagattcat atgaccctca gctttctgat 4680
   gtgtatgtat gaccttacac tgacactctg aactaatgta ggtatcttgt cctgcaggaa 4740
   ttcggcacga gtgtcgtcag gctccatatg atattgctta gcttccatat gatacaatac 4800
   tatcagtttg ctgcgtcatg gtctttgccc ctgctggtcc ttgttgcatg atcttgacac 4860
   atttggcctc ttttcgcagc ctcccgctgt gggctgtggc gatcctcacc ctcttccttg 4920
   atatcgacgg gatcggcaca ctcacctggg tttctttcat ccctctcatc atcctcttgt 4980
   gtgttggaac caagctagag atgatcatca tggagatggc cctggagatc caggaccggt 5040
   cgagcgtcat caagggggca cccgtggtcg agcccagcaa caagttcttc tggttccacc 5100
   gccccgactg ggtcctcttc ttcatacacc tgacgctgtt ccagaacgcg tttcagatgg 5160
   cacatttcgt gtggacaggc atgcgactgg gcatgcccgc tgaaatcacc agtctctctc 5220
   tacaaatcta tctctctcta taataatgtg tgagtagttc ccagataagg gaattagggt 5280
   tcttataggg tttcgctcat gtgttgagca tataagaaac ccttagtatg tatttgtatt 5340
   tgtaaaatac ttctatcaat aaaatttcta attcctaaaa ccaaaatcca ggggtaccga 5400
   gctcgaattc tagtctacgc ggccgcgagc tccagctttt gttcccttta gtgagggtta 5460
   attgcgcgct tggcgtaatc atggtcatag ctgtttcctg tgtgaaattg ttatccgctc 5520
   acaattccac acaacatacg agccggaagc ataaagtgta aagcctgggg tgcctaatga 5580
   gtgagctaac tcacattaat tgcgttgcgc tcactgcccg ctttccagtc gggaaacctg 5640
   tcgtgccagc tgcattaatg aatcggccaa cgcgcgggga gaggcggttt gcgtattggg 5700
   cgctcttccg cttcctcgct cactgactcg ctgcgctcgg tcgttcggct gcggcgagcg 5760
   gtatcagctc actcaaaggc ggtaatacgg ttatccacag aatcagggga taacgcagga 5820
   aagaacatgt gagcaaaagg ccagcaaaag gccaggaacc gtaaaaaggc cgcgttgctg 5880
   gcgtttttcc ataggctccg cccccctgac gagcatcaca aaaatcgacg ctcaagtcag 5940
   aggtggcgaa acccgacagg actataaaga taccaggcgt ttccccctgg aagctccctc 6000
   gtgcgctctc ctgttccgac cctgccgctt accggatacc tgtccgcctt tctcccttcg 6060
   ggaagcgtgg cgctttctca tagctcacgc tgtaggtatc tcagttcggt gtaggtcgtt 6120
   cgctccaagc tgggctgtgt gcacgaaccc cccgttcagc ccgaccgctg cgccttatcc 6180
   ggtaactatc gtcttgagtc caacccggta agacacgact tatcgccact ggcagcagcc 6240
   actggtaaca ggattagcag agcgaggtat gtaggcggtg ctacagagtt cttgaagtgg 6300
   tggcctaact acggctacac tagaaggaca gtatttggta tctgcgctct gctgaagcca 6360
   gttaccttcg gaaaaagagt tggtagctct tgatccggca aacaaaccac cgctggtagc 6420
   ggtggttttt ttgtttgcaa gcagcagatt acgcgcagaa aaaaaggatc tcaagaagat 6480
   cctttgatct tttctacggg gtctgacgct cagtggaacg aaaactcacg ttaagggatt 6540
   ttggtcatga gattatcaaa aaggatcttc acctagatcc ttttaaatta aaaatgaagt 6600
   tttaaatcaa tctaaagtat atatgagtaa acttggtctg acagttacca atgcttaatc 6660
   agtgaggcac ctatctcagc gatctgtcta tttcgttcat ccatagttgc ctgactcccc 6720
   gtcgtgtaga taactacgat acgggagggc ttaccatctg gccccagtgc tgcaatgata 6780
   ccgcgagacc cacgctcacc ggctccagat ttatcagcaa taaaccagcc agccggaagg 6840
   gccgagcgca gaagtggtcc tgcaacttta tccgcctcca tccagtctat taattgttgc 6900
   cgggaagcta gagtaagtag ttcgccagtt aatagtttgc gcaacgttgt tgccattgct 6960
   acaggcatcg tggtgtcacg ctcgtcgttt ggtatggctt cattcagctc cggttcccaa 7020
   cgatcaaggc gagttacatg atcccccatg ttgtgcaaaa aagcggttag ctccttcggt 7080
   cctccgatcg ttgtcagaag taagttggcc gcagtgttat cactcatggt tatggcagca 7140
   ctgcataatt ctcttactgt catgccatcc gtaagatgct tttctgtgac tggtgagtac 7200
   tcaaccaagt cattctgaga atagtgtatg cggcgaccga gttgctcttg cccggcgtca 7260
   atacgggata ataccgcgcc acatagcaga actttaaaag tgctcatcat tggaaaacgt 7320
   tcttcggggc gaaaactctc aaggatctta ccgctgttga gatccagttc gatgtaaccc 7380
   actcgtgcac ccaactgatc ttcagcatct tttactttca ccagcgtttc tgggtgagca 7440
   aaaacaggaa ggcaaaatgc cgcaaaaaag ggaataaggg cgacacggaa atgttgaata 7500
   ctcatactct tcctttttca atattattga agcatttatc agggttattg tctcatgagc 7560
   ggatacatat ttgaatgtat ttagaaaaat aaacaaatag gggttccgcg cacatttccc 7620
   cgaaaagtgc cac                                                    7633
<210> 10
   <211> 30
   <212> DNA
   <213> Künstliche Sequenz
<220>
   <223> Beschreibung der künstlichen Sequenz: Adaptor-Primer
<400> 10
   atatatctgc agggagccac ggccgtccac 30
<210> 11
   <211> 27
   <212> DNA
   <213> Künstliche Sequenz
<220>
   <223> Beschreibung der künstlichen Sequenz: Adaptor-Primer
<400> 11
   tatcccgggc ccgtgcctgg acgggaa 27
<210> 12
   <211> 30
   <212> DNA
   <213> Künstliche Sequenz
<220>
   <223> Beschreibung der künstlichen Sequenz: Adaptor-Primer
<400> 12
   atatatctcg agtctagaac tagtggatcc 30
<210> 13
   <211> 30
   <212> DNA
   <213> Künstliche Sequenz
<220>
   <223> Beschreibung der künstlichen Sequenz: Adaptor-Primer
<400> 13
   atatattacg tagtttgtcc gtgaacttca 30
<210> 14
   <211> 41
   <212> DNA
   <213> Künstliche Sequenz
<220>
   <223> Beschreibung der künstlichen Sequenz: Oligonukleotid
<400> 14
   gtacacaggc agctagctct cgaaacctcg ctcgaaacgc a 41
<210> 15
   <211> 41
   <212> DNA
   <213> Künstliche Sequenz
<220>
   <223> Beschreibung der künstlichen Sequenz: Oligonukleotid
<400> 15
   catgtgtccg tcgatcgaga gctttggagc gagctttgcg t 41

## Beschreibung

Die vorliegende Erfindung betrifft Promotorregionen, unter deren Kontrolle Transgene in Pflanzen epidermisspezifisch exprimiert werden können. Weiterhin betrifft die Erfindung rekombinante Nukleinsäuremoleküle, die diese Promotorregionen umfassen und transgene Pflanzen und Pflanzenzellen, die mit diesen Nukleinsäuremolekülen transformiert wurden, sowie Verfahren zu deren Herstellung. Außerdem betrifft die vorliegende Erfindung Nukleinsäuremoleküle umfassend einen erfindungsgemäßen Promotor und Nukleinsäuresequenzen bzw. Transgene, die Pathogenresistenz vermitteln können sowie mit diesen Nukleinsäuremolekülen transformierte Pflanzen und Pflanzenzellen und Verfahren zu deren Herstellung.

Als Promotoren werden allgemein diejenigen DNA-Bereiche eines Gens bezeichnet, die stromaufwärts des Transkriptionsstartpunktes liegen und durch die der Initiationspunkt und die Initiationshäufigkeit der Transkription und somit die Expressionsstärke und das Expressionsmuster des kontrollierten Gens festgelegt werden. An die Promotoren binden die RNA-Polymerase und spezifische, die RNA-Polymerase aktivierende Transkriptionsfaktoren, um die Transkription zusammen mit dem basalen Transkriptionskomplex zu initiieren. Die Wirksamkeit der Promotoren wird häufig durch zusätzliche DNA-Sequenzen, die Enhancer-Sequenzen, gesteigert und reguliert, deren Position im Gegensatz zu der der Promotoren nicht festgelegt ist. Diese regulatorischen Elemente können stromaufwärts, stromabwärts oder in einem Intron des zu exprimierenden Gens liegen.

In der rekombinanten DNA-Technologie werden Promotoren in Expressionsvektoren eingesetzt, um die Expression eines Transgens zu steuern, das in der Regel nicht das natürlicherweise durch den Promotor regulierte Gen ist. Dabei kommt es wesentlich auf die Spezifität des Promotors an, die bestimmt, zu welchem Zeitpunkt, in welchen Gewebetypen und in welcher Intensität ein gentechnisch übertragenes Gen exprimiert wird.

In der Pflanzenzucht wird die rekombinante DNA-Technologie häufig eingesetzt, um bestimmte nützliche Eigenschaften auf Nutzpflanzen zu übertragen, was zu einem höheren Ertrag, z. B. durch erhöhte Pathogenresistenz, oder zu verbesserten Eigenschaften der Ernteprodukte führen soll. Dabei ist es häufig wünschenswert, dass das übertragene Gen nicht ubiquitär exprimiert wird, sondern nur in den Geweben, in denen die Transgenaktivität gewünscht wird, da sich die Anwesenheit des Transgenprodukts in manchen Geweben negativ auf normale physiologische Prozesse auswirken kann. So konnte etwa gezeigt werden, dass die Überexpression einer anionischen Peroxidase unter der Kontrolle des ubiquitär wirkenden 35S-Promotors zum Welken von transgenen Tabakpflanzen führt, weil weniger Wurzelwachstum stattfindet und sich daher auch weniger Wurzelmasse bildet (Lagrimini et al. (1997) The consequence of peroxidase overexpression in transgenic plants on root growth and development. Plant Mol Biol. 33 (5), S. 887-895). Die Überexpression der spi2-Peroxidase unter der Kontrolle des ebenfalls ubiquitär wirkenden Ubiquitin-Promotors führt zu einer reduzierten Epicotylbildung und einem reduzierten Längenwachstum verglichen mit Kontrollpflanzen (Elfstrand, M. et al. (2001) Overexpression of the endogenous peroxidase-like gene spi 2 in transgenic Norway spruce plants results in increased total peroxidase activity and reduced growth. Plant Cell Reports 20 (7), S. 596-603). Abgesehen von negativen Effekten auf physiologische Prozesse soll in der Resistenzzüchtung häufig vermieden werden, dass das Transgenprodukt auch in den geernteten Pflanzenteilen vorliegt.

Deshalb wurden in den vergangenen Jahren Promotoren isoliert, die entweder gewebespezifisch oder induzierbar wirken. Zu den gewebespezifischen Promotoren gehören etwa samen-, knollen- und fruchtspezifische Promotoren. Die induzierbaren Promotoren können beispielsweise durch chemische Induktion, durch Lichtinduktion oder andere Stimuli aktiviert werden.

Es ist auch wünschenswert, die Genexpression spezifisch in der Epidermis zu modulieren. Die Epidermis stellt das Abschlussgewebe der oberirdischen Organe höherer Pflanzen dar. Als solches bestehen die Aufgaben der Epidermis darin, einerseits den Wasser- und Stoffaustausch der Pflanze zu ermöglichen und andererseits das Eindringen von Pathogenen in die Pflanze zu verhindern. Durch eine veränderte Genexpression in der Epidermis mit Hilfe geeigneter Promotoren und von ihnen gesteuerter Gene könnten diese Funktionen gezielt moduliert werden. In dikotyledonen Pflanzen wurden epidermisspezifische Promotoren bereits beschrieben. So konnte gezeigt werden, dass der Promotor des CER6- (CUT1-) Gens aus Arabidopsis, das für ein kondensierendes Enzym bei der Wachssynthese kodiert, die epidermisspezifische Expression eines β-Glucuronidase-Reportergens bewirken kann (Hooker et al. (2002), Significance of the expression of the CER6 condensing enzyme for cuticular wax production in Arabidopsis, Plant Physiol. 129(4), S. 1568-1580; Kunst et al. (2000), Expression of the wax-specific condensing enzyme CUT1 in Arabidopsis, Biochem. Soc. Trans. 28(6), S. 651-654).

Jedoch ist es bisher nicht gelungen, geeignete epidermisspezifische Promotoren in monokotyledonen Pflanzen, die sich für die Expression von Transgenen in Monokotyledonen, insbesondere Poaceen (Süßgräsern), besonders gut eignen, zu identifizieren. Deshalb wurden bisher konstitutive Promotoren wie der Ubiquitin-Promotor aus Mais verwendet, um Proteine in der Epidermis zu exprimieren (siehe z. B. Oldach et al. (2001), Heterologous expression of genes mediating enhanced fungal resistance in transgenic wheat, Mol Plant Microbe Interact. 14(7), S. 832-838). Dies kann aber, wie oben beschrieben, zu unerwünschten Nebeneffekten bei den transgenen Pflanzen aufgrund der Anwesenheit des Transgenprodukts in anderen Geweben bzw. Organen als der Epidermis führen.

Aufgabe der Erfindung ist es daher, Mittel bereitzustellen, die eine epidermisspezifische Genexpression in Monokotyledonen, bevorzugt in Getreidepflanzen, ermöglichen.

Diese Aufgabe wird gelöst durch die Bereitstellung der in den Patentansprüchen charakterisierten Ausführungsformen.

Somit betrifft die vorliegende Erfindung eine Promotorregion mit Spezifität für die pflanzliche Epidermis, umfassend eine erste, aus dem Promotor des Gens Glutathion-S-Transferase A1 (GSTA1) stammende Sequenz, und eine zweite, aus dem Intron des Gens WIR1a stammende Sequenz. GSTA1 bezieht sich auf Gene, wie sie in Dudler et al. (1991), A pathogen-induced wheat gene encodes a protein homologous to glutathione-S-transferases, Mol. Plant Microbe Interact. 4(1), S. 14-18 beschrieben sind. Insbesondere handelt es sich bei diesen Genen um Gene aus Weizen, es kann sich aber auch um homologe Gene aus anderen Getreidepflanzen, vor allem Gerste, mit vergleichbarem Expressionsmuster und ähnlichem Genprodukt handeln. WIR1a bezeichnet Gene, wie sie in Bull et al. (1992), Sequence and expression of a wheat gene that encodes a novel protein associated with pathogen defense, Mol. Plant Microbe Interact. 5(6), S. 516-519, beschrieben sind.

Bevorzugt handelt es sich bei der ersten Sequenz um SEQ ID Nr. 1 und bei der zweiten Sequenz um SEQ ID Nr. 2.

Zwischen der ersten und der zweiten Sequenz können weitere, untranslatierte Sequenzen liegen, die eine Länge von 10bp bis 1000bp, bevorzugt von 20bp bis 800bp, besonders bevorzugt von 30bp bis 500bp und am meisten bevorzugt zwischen 40bp und 300bp aufweisen.

Besonders bevorzugt handelt es sich bei der erfindungsgemäßen Promotorregion um eine Promotorregion, ausgewählt aus der Gruppe bestehend aus
a) Promotorregionen, die die unter SEQ ID Nr. 3 angegebene Nukleinsäuresequenz umfassen;
b) Promotorregionen, die einen funktionalen Teil der unter SEQ ID Nr. 3 angegebenen Nukleinsäuresequenz umfassen oder
c) Promotorregionen, die eine Sequenz aufweisen, die unter stringenten Bedingungen
mit der unter SEQ ID Nr. 3 angegebenen Nukleinsäuresequenz hybridisiert.

Im Rahmen der vorliegenden Erfindung wird unter einer Promotorregion eine Nukleinsäuresequenz verstanden, die die zur Expression einer kodierenden Sequenz (Transgen) notwendigen regulatorischen Sequenzen umfasst. Regulatorische Sequenzen bilden denjenigen Teil eines Gens, der die Expression einer kodierenden Sequenz bestimmt, also vor allem das Expressionsniveau und -muster. Die regulatorischen Sequenzen besitzen mindestens ein Sequenzmotiv, an das spezifische Transkriptionsfaktoren und die RNA-Polymerase binden, zum Transkriptionskomplex assemblieren und die Transkription der von der Promötorregion kontrollierten Nukleinsäuresequenz wirksam initiieren.

Die erfindungsgemäßen Promotorregionen basieren auf der Beobachtung, dass durch Fusion des Promotors des GSTA1-Gens aus Weizen mit intronischen Sequenzen des WIR1a-Gens aus Weizen Promotoren mit neuen Eigenschaften hergestellt werden können.

In transienten Reportergenassays in Weizenblättern mit einem β-Glucuronidase-(GUS)-Gen aus *E. coli* als Reportergen wurden verschiedene Kombinationen des WIR1a-Promotors und -Introns und des GST-Promotors getestet. Es zeigte sich überraschenderweise, dass GST-Promotor und WIR1a-Intron einen synergistischen Effekt auf die Reportergen-Aktivität ausüben. Die Steigerung der transkriptionellen Aktivität war vergleichbar mit der durch den ubiquitär exprimierten 35S-Promotor erzielten transkriptionellen Aktivität.

Unter dem Begriff "epidermisspezifisch" wird im Rahmen der vorliegenden Erfindung verstanden, dass eine unter der Kontrolle der erfindungsgemäßen Promotorregion stehende Nukleinsäuresequenz in der Sprossepidermis von Pflanzen exprimiert wird. Insbesondere ist Epidermisspezifität im Sinne der vorliegenden Erfindung auch dann gegeben, wenn die erfindungsgemäße Promotorregion die Expression eines Fremdgens in der Epidermis im Vergleich zu anderen Zelltypen begünstigt und in der Epidermis eine signifikant, wie mindestens 2-fach, bevorzugt mindestens 5- fach und besonders bevorzugt mindestens 10- und am meisten bevorzugt 50-fach gegenüber anderen Zelltypen erhöhte Expression bewirkt. Die Expressionshöhe kann mit üblichen in situ-Nachweistechniken bestimmt werden.

Der Begriff "pflanzliche Epidermis" ist dem Fachmann geläufig. Ergänzende Informationen sind in jedem Pflanzenanatomie- oder -physiologiebuch zu finden, wie etwa in Strasburger, Lehrbuch der Botanik, 35. Auflage 2002, Spektrum Akademischer Verlag.

Es wurde nun überraschenderweise gefunden, dass eine Promotorregion, die sowohl regulatorische Sequenzen aus dem GSTA1-Gen aus Weizen als auch Intron-Sequenzen aus dem WIR1a-Gen aus Weizen umfasst, eine epidermisspezifische Expression einer unter ihrer Kontrolle stehenden kodierenden Nukleinsäuresequenz bewirkt.

Neben einer Promotorregion, die die unter SEQ ID Nr. 3 dargestellten Nukleinsäuresequenzen aufweist, betrifft die vorliegende Erfindung auch Promotorregionen, die die funktionalen Teile dieser Sequenz aufweisen und die in Pflanzen eine epidermisspezifische Expression einer von ihnen kontrollierten kodierenden Nukleinsäuresequenz bewirken.

Unter einem "funktionalen Teil" werden in diesem Zusammenhang Sequenzen verstanden, an die der Transkriptionskomplex trotz leicht abweichender Nukleinsäuresequenz noch binden und epidermisspezifische Expression bewirken kann. Funktionale Teile einer Promotorsequenz umfassen auch solche Promotorvarianten, deren Promotoraktivität, verglichen mit dem Wildtyp, abgeschwächt oder verstärkt ist. Unter einem funktionalen Teil versteht man insbesondere auch natürliche oder künstliche Varianten der in SEQ ID Nr. 3 angegebenen Sequenz der Promotorregion. Mutationen umfassen Substitutionen, Additionen, Deletionen, Vertauschungen und/oder Insertionen eines oder mehrerer Nukleotidreste. Funktionale Teile der Promotorregionen umfassen im Rahmen der vorliegenden Erfindung natürlich vorkommende Varianten der SEQ ID Nr. 3 sowie künstliche, z. B. durch chemische Synthese erhaltene Nukleotidsequenzen.

Der verwendete Promotor enthält in jedem Fall eine TATA-Box (Positionen 2163 bis 2169 in SEQ ID Nrn. 1 und 3) und bevorzugt auch zwei CAAT-Boxen (Positionen 1047 bis 1051 bzw. 1895 bis 1899 in SEQ ID Nrn. 1 und 3). Weiterhin ist mindestens eines, bevorzugt mindestens zwei und drei, besonders bevorzugt mindestens vier, fünf und sechs, und am meisten bevorzugt sieben und acht der folgenden Sequenzmotive im Promotor enthalten:
a) GTGGGGG
b) ACGTGGA
c) TCCACCT
d) TATCCAT
e) CATGCATG
f) TGTAAAG
g) CCTACCA
h) AATAGTA

Bevorzugt liegen die Sequenzmotive an den Positionen, die den folgenden Positionen in SEQ ID Nrn. 1 und 3 entsprechen:
- a) 185-191 und 217-223bp
- b)455-461bp
- c) 508-514bp
- d) 564-570bp
- e) 1514-1521bp
- f) 1520-1526bp
- g) 1569-1575bp
- h) 1610-1616bp

Gemessen werden kann die Promotoraktivität von Varianten der Promotorregion mit Hilfe von Markergenen, deren kodierende Sequenz unter der Kontrolle der zu untersuchenden Promotorregion steht. Geeignete Markergene sind beispielsweise das β-Glucuronidase-(GUS)-Gen aus *E. coli,* ein Fluoreszenzgen wie etwa das Green-Fluorescence-Protein (GFP)-Gen aus *Aequoria victoria,* das Luziferase-Gen aus *Photinus pyralis* oder das β-Galaktosidase-(lacZ)-Gen aus *E. coli.* Die absolute Promotoraktivität wird bestimmt durch Vergleich mit einer Wildtyp-Pflanze. Die Gewebe- bzw. Zellspezifität lässt sich leicht durch Vergleich der Expressionsraten der oben genannten Markergene in den jeweiligen Geweben bzw. Zellen bestimmen.

Die vorliegende Erfindung betrifft ebenfalls Promotorregionen mit einer Nukleinsäuresequenz, die mit der unter SEQ ID Nr. 3 angegebenen Nukleinsäuresequenz unter stringenten Bedingungen hybridisiert. Der Begriff "Hybridisierung unter stringenten Bedingungen" bedeutet im Zusammenhang dieser Erfindung, dass die Hybridisierung *in vitro* unter Bedingungen durchgeführt wird, die stringent genug sind, um eine spezifische Hybridisierung zu gewährleisten. Solche stringenten Hybridisierungsbedingungen sind dem Fachmann bekannt und können der Literatur entnommen werden (Sambrook et al. (2001), Molecular Cloning: A Laboratory Manual, 3. Auflage, Cold Spring Harbor Laboratory Press, Cold Spring Harbor, New York).

Allgemein bedeutet "spezifisch hybridisieren", dass ein Molekül unter stringenten Bedingungen präferenziell an eine bestimmte Nukleotidsequenz bindet, wenn diese Sequenz in einem komplexen Gemisch von (z. B. Gesamt-) DNA oder RNA vorliegt. Der Begriff "stringente Bedingungen" steht allgemein für Bedingungen, unter denen eine Nukleinsäuresequenz präferenziell an ihre Zielsequenz hybridisieren wird, und zu einem deutlich geringeren Ausmaß oder gar nicht an andere Sequenzen. Stringente Bedingungen sind z. T. Sequenz-abhängig und werden unter verschiedenen Umständen unterschiedlich sein. Längere Sequenzen hybridisieren spezifisch bei höheren Temperaturen. Im Allgemeinen werden stringente Bedingungen so ausgewählt, dass die Temperatur etwa 5°C unter dem thermischen Schmelzpunkt (Tₘ) für die spezifische Sequenz bei einer definierten Ionenstärke und einem definierten pH liegt. Die Tₘ ist die Temperatur (unter definierter Ionenstärke, pH und Nukleinsäurekonzentration), bei der 50% der zu der Zielsequenz komplementären Moleküle zu der Zielsequenz im Gleichgewichtszustand hybridisieren. Typischerweise sind stringente Bedingungen solche, bei denen die Salzkonzentration mindestens ungefähr 0,01 bis 1,0 M Natriumionen-Konzentration (oder ein anderes Salz) bei einem pH zwischen 7,0 und 8,3 beträgt und die Temperatur mindestens 30 °C für kurze Moleküle (also z. B. 10-50 Nukleotide) beträgt. Zusätzlich können stringente Bedingungen durch Zugabe destabilisierender Agenzien, wie beispielsweise Formamid, erreicht werden.

Geeignete stringente Hybridisierungsbedingungen sind beispielsweise auch beschrieben in Sambrook et al.,vide supra. So kann die Hybridisierung etwa unter den folgenden Bedingungen stattfinden:
- Hybridisierungspuffer: 2x SSC, 10x Denhardt's Lösung (Fikoll 400 + PEG + BSA; Verhältnis 1:1:1), 0,1% SDS, 5mM EDTA, 50mM Na₂HPO₄, 250µg/ml Heringssperma-DNA; 50µg/ml tRNA oder 0,25M Natriumphosphatpuffer pH 7,2, 1mM EDTA, 7% SDS bei einer Hybridisierungstemperatur von 65°C bis 68°C
- Waschpuffer: 0,2x SSC, 0,1% SDS bei einer Waschtemperatur von 65°C bis 68°C

Vorzugsweise weisen derartige Promotorvarianten eine Sequenzidentität von mindestens 50%, bevorzugt mindestens 70%, besonders bevorzugt mindestens 90% und am meisten bevorzugt mindestens 95% zu der unter SEQ ID Nr. 3 angegebenen Promotorsequenz oder Teilen davon auf, bezogen auf die gesamte in SEQ ID Nr. 3 gezeigte DNA-Sequenz. Vorzugsweise wird die Sequenzidentität derartiger Promotorsequenzen durch Vergleich mit der unter SEQ ID Nr. 3 angegebenen Nukleinsäuresequenz bestimmt. Wenn zwei unterschiedlich lange Nukleinsäuresequenzen miteinander verglichen werden, bezieht sich die Sequenzidentität vorzugsweise auf den prozentualen Anteil der Nukleotidreste der kürzeren Sequenz, die identisch sind mit den entsprechenden Nukleotidresten der längeren Sequenz.

Sequenzidentitäten werden üblicherweise über verschiedene Alignment-Programme, wie z. B. CLUSTAL festgestellt. Allgemein stehen dem Fachmann zur Bestimmung der Sequenzidentität geeignete Algorithmen zur Verfügung, z. B. auch das Programm, das unter http://www.ncbi.nlm.nih.gov/BLAST (z. B. der Link "Standard nucleotide-nucleotide BLAST [blastn]") zugänglich ist.

Die oben für SEQ ID Nr. 3 angegebenen prozentualen Identitätsgrade gelten ebenso für die in SEQ ID Nrn. 1 und 2 gezeigten ersten und zweiten Sequenzen der erfindungsgemäßen Promotorregion.

In einer bevorzugten Ausführungsform der Erfindung weist die erfindungsgemäße Promotorregion die gesamte unter SEQ ID Nr. 3 angegebene Sequenz von 2552 Nukleotiden auf.

Die vorliegende Erfindung betrifft auch chimäre Gene aus der erfindungsgemäßen Promotorregion und einer kodierenden Sequenz, deren Expression, die natürlicherweise nicht durch die erfindungsgemäßen Promotorregion reguliert wird, im chimären Gen durch die erfindungsgemäße Promotorregion reguliert wird, in operativer Verknüpfung sowie rekombinante Nukleinsäuremoleküle, die diese chimären Gene enthalten.

Der Begriff "Nukleinsäuresequenz, deren Expression durch die erfindungsgemäße Promotorregion reguliert wird" bedeutet, dass die Expression der Nukleinsäuresequenz unter der Kontrolle der erfindungsgemäßen Promotorregion in den Zellen, in denen die Promotorregion aktiv ist, um mindestens den Faktor fünf, bevorzugt mindestens den Faktor 10 und besonders bevorzugt mindestens den Faktor 50 gegenüber Wildtyp-Zellen gesteigert werden kann.

Bei der Nukleinsäuresequenz, deren Expression durch die erfindungsgemäße Nukleinsäuresequenz reguliert wird, kann es sich um die kodierende Region eines Transgens handeln, z. B. eines Resistenzgens, dessen Genprodukt in der Epidermis erwünscht ist. Durch die Expression des Transgens kann der Gehalt des von ihm kodierten Genprodukts mindestens um den Faktor 2, bevorzugt mindestens um den Faktor 5, besonders bevorzugt mindestens um den Faktor 10 und am meisten bevorzugt mindestens um den Faktor 50 erhöht werden.

Die erfindungsgemäße Promotorregion kann aber auch in RNAi-Konstrukten zur RNA-Interferenz eingesetzt werden, um das epidermisspezifische Silencing bestimmter Gene zu erreichen, deren Genprodukte in der Epidermis nicht oder in geringerem Ausmaß als üblich anwesend sein sollen. Letzteres kann natürlich auch mit klassischen antisense- oder Kosuppressionskonstrukten unter Einsatz der erfindungsgemäßen Promotorregionen erreicht werden. Die Expression des endogenen Gens wird durch die Silencing-Konstrukte um mindestens 50%, bevorzugt um mindestens 70%, besonders bevorzugt um mindestens 90% und besonders bevorzugt um mindestens 95% verringert.

In einem Konstrukt, das zur RNA-Interferenz verwendet werden soll, liegen üblicherweise palindromische DNA-Sequenzen vor, die nach der Transkription doppelsträngige RNA bilden. Diese doppelsträngige RNA wird durch das Dicer-Enzym zu kürzeren RNA-Stücken prozessiert, die an eine endogene RNA binden und deren Abbau mit Hilfe des RISC (RNA-induced silencing complex) bewirken (Hannon (2002) RNA interference, Nature, Bd. 418, S. 244-251).

Der Effekt der Gen-Silencing-Konstrukte auf die Expression des endogenen Gens kann mit Hilfe gängiger molekularbiologischer Methoden nachgewiesen werden, die dem Fachmann wohl bekannt sind. So stehen zur Untersuchung des RNA-Levels Northern-Blot- und RT-PCR-Verfahren zur Verfügung, das Protein kann durch Western-Blot-Analysen, Immunfluoreszenzen oder, sofern es sich bei dem Protein um ein Enzym handelt, Enzymassays nachge-wiesen werden.

Unter dem Begriff "Transgen" werden im Rahmen der vorliegenden Erfindung diejenigen Gene zusammengefasst, deren Genprodukte in der Epidermis bereitgestellt werden sollen, bzw. beim Gen-Silencing unterdrückt werden sollen.

Bei der Nukleinsäuresequenz, deren Expression unter der Kontrolle des erfindungsgemäßen Promotors steht, handelt es sich bevorzugt um eine Nukleinsäuresequenz, die Pathogenresistenz vermittelt, da die Epidermis die erste Barriere darstellt, die von einem Pathogen beim Eindringen in die Pflanze überwunden werden muss.

Im Rahmen der vorliegenden Erfindung wird unter dem Begriff "rekombinantes Nukleinsäuremolekül" ein Vektor verstanden, der ein erfindungsgemäßes chimäres Gen oder eine erfindungsgemäße Promotorregion enthält und die promotorabhängige Expression der unter der Kontrolle der erfindungsgemäßen Promotorregion stehenden Nukleinsäuresequenz in Pflanzenzellen und Pflanzen bewirken kann. In einer bevorzugten Ausführungsform enthält ein erfindungsgemäßes rekombinantes Nukleinsäuremolekül zusätzlich transkriptionelle Terminationssequenzen. Unter "transkriptionellen Terminationssequenzen" werden dabei DNA-Sequenzen verstanden, die am Stromabwärts-Ende einer kodierenden Sequenz liegen und die RNA-Polymerase zum Stoppen der Transkription veranlassen.

Weiterhin betrifft die Erfindung Verfahren zur Herstellung transgener Pflanzen mit epidermisspezifischer Expression einer durch die erfindungsgemäße Promotorregion regulierten Nukleinsäuresequenz, umfassend die Schritte:
a) Herstellung eines rekombinanten Nukleinsäuremoleküls, in der die erfindungsgemäße Promotorregion in operativer Verknüpfung mit einer kodierenden Sequenz vorliegt,
b) Übertragung des Nukleinsäuremoleküls aus a) auf pflanzliche Zellen und
c) Regeneration vollständig transformierter Pflanzen und, falls erwünscht, Vermehrung der Pflanzen.

Zur Vorbereitung der Einführung fremder Gene in höhere Pflanzen bzw. deren Zellen stehen eine große Anzahl von Klonierungsvektoren zur Verfügung, die ein Replikationssignal für *E. coli* und ein Markergen zur Selektion transformierter Bakterienzellen enthalten. Beispiele für derartige Vektoren sind pBR322, pUC-Serien, M13mp-Serien, pACYC184 usw. Das chimäre Gen kann an einer passenden Restriktionsschnittstelle in den Vektor eingeführt werden. Das erhaltene Plasmid wird dann für die Transformation von *E. coli*-Zellen verwendet. Transformierte *E. coli*-Zellen werden in einem geeigneten Medium gezüchtet und anschließend geerntet und lysiert, und das Plasmid wird wiedergewonnen. Als Analysemethoden zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und daraus gewonnene DNA-Fragmente mit anderen DNA-Sequenzen verknüpft werden.

Wie bereits erwähnt, stehen für die Einführung von DNA in eine pflanzliche Wirtszelle eine Vielzahl von Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmedium, die Fusion von Protoplasten, die Injektion, die Elektroporation, den direkten Gentransfer isolierter DNA in Protoplasten, die Einbringung von DNA mittels biolistischer Methoden sowie weitere Möglichkeiten, die bereits seit mehreren Jahren gut etabliert sind und zum üblichen Repertoire des Fachmanns in der pflanzlichen Molekularbiologie bzw. Pflanzenbiotechnologie gehören. Die biolistische Gentransfermethode wird vor allem bei monokotyledonen Pflanzen verwendet. Hier findet der Fachmann nützliche Informationen zur Durchführung z.B. in Vasil et al. (1992) Bio/Technology, 10, S. 667-674; Vasil et al. (1993) Bio/Technology, 11, S. 1153-1158; Nehra et al. (1994) Plant J. 5, S. 285-297; Becker et al. (1994) Plant J., 5, S. 299-307; Altpeter et al. (1996) Plant Cell Reports 16, S. 12-17; Ortiz et al. (1996) Plant Cell Reports 15, S. 877-81; Rasco-Gaunt et al. (2001) J. Exp. Bot. 52; S. 865-874.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide, wie z.B. pUC-Derivate verwendet werden.

Sollen aber aus derartig transformierten Zellen ganze Pflanzen regeneriert werden, ist die Anwesenheit eines selektierbaren Markergens empfehlenswert. Dem Fachmann sind die gängigen Selektionsmarker bekannt, und es stellt für ihn kein Problem dar, einen geeigneten Marker auszuwählen.

Je nach Einführungsmethode der gewünschten Gene in die Pflanzenzelle können weitere DNA-Sequenzen erforderlich sein. Wird z.B. zur Transformation der Pflanzenzelle das Ti- oder Ri-Plasmid verwendet, so muss mindestens die rechte Begrenzung, häufig jedoch die rechte und linke Begrenzung der im Ti- bzw. Ri-Plasmid enthaltenen T-DNA als Flankenbereich mit den einzuführenden Genen verbunden werden. Werden zur Transformation Agrobakterien verwendet, muss die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige *vir*-Region. Intermediäre Vektoren können allerdings nicht in Agrobakterien replizieren. Mittels eines Helferplasmids kann der intermediäre Vektor auf *Agrobacterium tumefaciens* übertragen werden (Konjugation). Binäre Vektoren dagegen können sowohl in *E. coli* als auch in Agrobakterien replizieren. Sie enthalten ein Selektionsmarkergen und einen Linker oder Polylinker, welche von der rechten und linken T-DNA-Grenzregion eingerahmt werden. Sie können direkt in die Agrobakterien transformiert werden. Das als Wirtszelle dienende Agrobakterium soll ein Plasmid enthalten, welches das chimäre Gen innerhalb der T-DNA trägt, welche in die Pflanzenzelle übertragen wird. Zusätzliche T-DNA kann vorhanden sein. Das derartig transformierte Agrobakterium wird zur Transformation von Pflanzenzellen verwendet. Die Verwendung von T-DNA für die Transformation von Pflanzenzellen ist intensiv untersucht und ausreichend in allseits bekannten Übersichtsartikeln und Handbüchern zur Pflanzentransformation beschrieben worden. Für monokotyledone Pflanzen müssen für einen effektiven Agrobakteriumvermittelten Gentransfer abgewandelte Protokolle angewandt werden, wie sie etwa in Cheng et al. (1997) Plant Physiol. 115, S. 971-980; Khanna and Daggard (2003) Plant Cell Reports 21, S. 429-436; Wu et al. (2003) Plant Cell Reports 21, S. 659-668; Hu et al. (2003) Plant Cell Reports 21, S. 1010-1019, beschrieben sind. Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin, Methotrexat, Glyphosat, Streptomycin, Sulfonylharnstoff, Gentamycin oder Phosphinotricin u.a. vermittelt. Der individuell gewählte Marker sollte daher die Selektion transformierter Zellen gegenüber Zellen, denen die eingeführte DNA fehlt, gestatten. Hierzu sind auch alternative Marker geeignet, wie nutritive Marker oder Screeningmarker (wie GFP, green fluorescent protein). Selbstverständlich kann auch vollkommen auf Selektionsmarker verzichtet werden, was allerdings mit einem ziemlich hohen Screeningbedarf einhergeht. Falls markerfreie transgene Pflanzen erwünscht sind, stehen dem Fachmann auch Strategien zur Verfügung, die eine nachträgliche Entfernung des Markergens erlauben, z.B. Cotransformation oder Sequenzspezifische Rekombinasen.

Die Regeneration der transgenen Pflanzen aus transgenen Pflanzenzellen erfolgt nach üblichen Regenerationsmethoden unter Verwendung bekannter Nährmedien. Die so erhaltenen Pflanzen können dann mittels üblicher Verfahren, einschließlich molekularbiologischer Methoden, wie PCR, Blot-Analysen, auf Anwesenheit und Gewebespezifität der eingeführten Nukleinsäuresequenz, deren Expression von dem erfindungsgemäßen Promotor kontrolliert wird, bzw. der von ihr beeinflussten endogenen RNAs und Proteine untersucht werden.

Ferner betrifft die Erfindung transgene Pflanzen, die eine durch die erfindungsgemäße Promotorregion regulierte Nukleinsäuresequenz enthalten und diese epidermisspezifisch exprimieren.

Bei den erfindungsgemäßen Pflanzen handelt es sich bevorzugt um Monokotyledonen, insbesondere Getreidepflanzen wie Roggen, Mais und Hafer, besonders bevorzugt um Weizen oder Gerste, sowie transgene Teile dieser Pflanzen und deren transgenes Vermehrungsmaterial, wie Protoplasten, Pflanzenzellen, Kalli, Samen, Knollen oder Stecklinge, sowie die transgenen Nachkommen dieser Pflanze. Aber auch für andere Poaceen (Süßgräser) wie z. B. Futtergräser kann die erfindungsgemäße Promotorregion zur Herstellung entsprechender Pflanzen mit epidermisspezifischer Expression von Transgenen eingesetzt werden.

Unter der Kontrolle des erfindungsgemäßen, epidermisspezifischen Promotors können Gene für die Produktion epikutikulärer Wachse exprimiert werden, um die Trockenheitstoleranz der Pflanzen zu erhöhen. Außerdem können unter der Kontrolle des erfindungsgemäßen Promotors Gene für die Produktion von Anthocyanen oder anderen UV-absorbierenden Substanzen zur Erhöhung der UV-Resistenz exprimiert werden. Wie oben bereits ausgeführt, werden bevorzugt Pathogen-Resistenzgene unter der Kontrolle des erfindungsgemäßen Promotors exprimiert.

Als Pflanzenpathogene werden unter anderem Bakterien, Viren und Pilze bezeichnet, die Pflanzen infizieren und dadurch den Stoffwechsel der Pflanze negativ beeinflussen.

Zu diesen Pflanzenpathogenen gehören Pilze, die bei Getreidepflanzen wie Weizen und Gerste unter anderem die Krankheiten Echter Mehltau und Halmbruchkrankheit auslösen. Diese Krankheiten können abhängig von der Befallsstärke erhebliche Ertragsverluste (bis zu 50%) verursachen.

Traditionell werden die oben genannten sowie weitere pflanzliche Pilzerkrankungen durch den Einsatz von Fungiziden bekämpft, die die bekannten Nachteile, wie Grundwassergängigkeit und Akkumulation in der Nahrungskette, besitzen.

In den letzten Jahren wurden aber auch einige Gene identifiziert, die Resistenz gegen einen bestimmten oder gegen mehrere Erreger vermitteln können. Der Begriff "Vermittlung von Pathogenresistenz", wie er hier verwendet wird, bedeutet, dass Pflanzen, in denen die Expression der besagten Gene erhöht ist, gegenüber Pflanzen, in denen die Expression der besagten Gene normal ist, weniger empfänglich für die Infektion mit bestimmten Pathogenen sind. Zu den Genen, die Pathogenabwehr vermitteln, gehören auch solche Gene, deren Expression durch Infektion mit einem Pathogen angeschaltet wird.

Zu diesen Genen gehören Peroxidasen und Oxalat-Oxidasen. Die Oxalat-Oxidasen, die zu der Familie der germinartigen Proteine gehören, katalysieren die Oxidation von Oxalat, wodurch Wasserstoffperoxid entsteht. Das Wasserstoffperoxid wirkt mikrobizid und kann die Lignifizierung der Zellwände fördern, wodurch das Eindringen von Schädlingen verhindert wird. Außerdem kann es in geringen Konzentrationen hypersensitiven Zelltod hervorrufen. Die Peroxidasen verwenden entweder molekularen Sauerstoff oder Wasserstoffperoxid, um zelluläre Substrate zu oxidieren und dadurch zu entgiften.

Pathogene, gegenüber denen die Expression der Oxalat-Oxidasen und Peroxidasen in der Epidermis von Pflanzen Resistenz vermitteln kann, schließen zum Beispiel ein: Echter Mehltau, Fusarium spp., Rynchosporium secalis und Pyrenophora teres.

Weitere Gene, die in der Lage sind, Resistenz gegen Pathogene zu vermitteln, sind Chitinasen, Ag-AFP, GSTA1 und WIR1a.

Durch die Expression der für diese Enzyme kodierenden Nukleinsäuresequenz in der Epidermis von transgenen Pflanzen mit Hilfe der erfindungsgemäßen Promotorregion können Pflanzen mit erhöhter Pathogenresistenz erhalten werden.

Im Gegensatz zu den Pathogenresistenz vermittelnden Genen gibt es auch pflanzeneigene Gene, die das Eindringen eines Pathogens fördern. Zu diesen gehört das Mlo-Gen, das für einen Sieben-Transmembran-Rezeptor kodiert, der das Eindringen des Mehltau-Pilzes in die Epidermis zu fördern scheint. In diesem Fall ist es sinnvoll, mit der Expression des Mlo-Gens zu interferieren, um das Eindringen von Pilzen in die Pflanze zu verhindern. Dies kann z. B. mit Hilfe der oben beschriebenen RNAi-Methode erfolgen. Dass die Interferenz mit der Expression des Mlo-Gens geeignet ist, das Eindringen des Mehltaupilzes in die Pflanze zu verhindern, wurde in vitro an Blattsegmenten aus Gerste gezeigt, die mit Wolfram-Teilchen beschossen wurden, die mit Mlo-dsRNA beschichtet worden waren (Schweizer et al. (2000), Double-stranded RNA interferes with gene function at the single-cell level in cereals, The Plant Journal, 24 (6), S. 895-903). Jedoch konnte bisher nicht gezeigt werden, dass die epidermis-spezifische Interferenz mit der Mlo-Expression in transgenen Pflanzen den gleichen Effekt hat.

Weitere pflanzliche Gene, die die Interaktion eines Pathogens mit der Pflanze vermitteln und dadurch das Eindringen des Pathogens in die Pflanze fördern können, sind beispielsweise Aminosäuren- oder Zuckertransporter oder Invertasen. Diese Gene eignen sich ebenfalls als Angriffspunkte für das Gen-Silencing. Somit betrifft die vorliegende Erfindung Verfahren zur Herstellung pathogenresistenter Pflanzen, umfassend die Schritte:
a) Herstellung eines rekombinanten Nukleinsäuremoleküls, in dem der erfindungsgemäße Promotor in operativer Verknüpfung mit einer Nukleinsäuresequenz, die Pathogenresistenz vermittelt, vorliegt,
b) Übertragung des rekombinanten Nukleinsäuremoleküls aus a) auf pflanzliche Zellen und
c) Regeneration vollständig transformierter Pflanzen und, falls erwünscht, Vermehrung der Pflanzen.

Bevorzugt handelt es sich bei der Pathogenresistenz vermittelnden Nukleinsäuresequenz um die kodierende Region eines Peroxidase- oder Oxalat-Oxidase-Gens oder um eine Sequenz, die mit der endogenen Mlo-RNA interferiert.

Die nachfolgenden Beispiele dienen zur Erläuterung der Erfindung und sollten nicht einschränkend verstanden werden.

### Abbildungen:

1) Nukleinsäuresequenz des GSTA1-Promotors (SEQ ID Nr. 1)
2) Nukleinsäuresequenz des WIR1a-Introns (SEQ ID Nr. 2)
3) Nukleinsäuresequenz der bevorzugten Promotorregion (SEQ ID Nr. 3)
4) Nukleinsäuresequenz der TAPERO (Peroxidase) cDNA (SEQ ID Nr. 4)
5) TAPERO-Expressionsvektor pPS41
   a) Nukleinsäuresequenz (SEQ ID Nr. 5)
   b) Vektorkarte
6) Nukleinsäuresequenz der Germin 9f-2.8 (Oxalat-Oxidase) cDNA (SEQ ID Nr. 6)
7) Germin-Expressionsvektor pPS24
   a) Nukleinsäuresequenz (SEQ ID Nr. 7)
   b) Vektorkarte
8) Sequenz des Mlo-RNAi-Konstrukts (SEQ ID Nr. 8)
9) Mlo-RNAi-Expressionsvektor pWIR5-TaMlo-RNAi
   a) Nukleinsäuresequenz (SEQ ID Nr. 9)
   b) Vektorkarte
10) *In situ* Oxalatoxidase-Aktivität in pPS24-transgenen Pflanzen Blätter von Bobwhite Wildtyppflanzen (B W) und von transgenen Linien Nr.157 und Nr. 170 wurden quergeschnitten und die Oxalatoxidase-Aktivität *in situ* nachgewiesen. Linke Spalte = Reaktion mit Oxalat-Substrat; rechte Spalte = Kontrollreaktion ohne Oxalat-Substrat. Die starke Violettfärbung zeigt Oxalatoxidase-Aktivität in der Epidermis der transgenen Linien an.
11) Nachweis des TAPERO-Transgens in pPS41-transgenen Pflanzen
   a) im Northern Blot
      Nachweis der Akkumulation von TAPERO RNA mittels Hybridisierung einer WIR3 Probe an Northern blots aus transgenen Weizenlinien der T2 Generation, die das pPS41 Konstrukt tragen. Es wurden je 2 Sublinien von 4 ausgewählten Linien plus Wildtyp (BW) im Adultpflanzenstadium analysiert. Blatt 1 = Fahnenblatt. Blätter 2-4 = zunehmend älter. Die TaGer-4 Sonde hybridisiert an eine Gruppe von stressinduzierten Weizengenen und wurde verwendet, um pleiotrope Nebeneffekte der TAPERO-Überexpression zu testen. Keine signifikante Nebenwirkung wurde gefunden. EtBr = Beladungskontrolle der Gele, gefärbt mit Ethidiumbromid.
   b) im Western Blot
      Nachweis der Akkumulation des TAPERO-Proteins mittels Antikörperreaktion auf Western Blots von transgenen Weizenlinien der T2 Generation, die das pPS41 Konstrukt tragen. Das TAPERO-Transgenprodukt hat die erwartete Grösse von 31 kD. In Bobwhite, Blatt 3 ist eine erhöhte Basalaktivität des TAPERO Gens zu beobachten. Blatt 1 = Fahnenblatt. Coomassie stain = Ladungskontrolle der Gele, gefärbt mit Coomassie Blau R250.
12) Nachweis der epidermisspezifischen Transgenexpression
   A) durch Northern Blot-Analyse
      Nachweis der Anhäufung von Oxalatoxidase- (links) und TaPERO (rechts)-mRNA in der Blattepidermis von transgenen Pflanzen, die das pPS24- bzw. das pPS41-Konstrukt tragen, mit spezifischen Sonden. W = RNA aus ganzem Blatt; E = RNA aus Blattepidermis. EtBr = Ethidiumbromid gefärbtes Gel als Ladungskontrolle; 26S RNA = Nachhybridisierung des Blots mit einer Sonde gegen die 26S ribosomale RNA als Ladungskontrolle.
   B) durch Real-time reverse PCR-Analyse
      Es wurde die Konzentration der TaPERO mRNA in Gesamtblatt und Epidermis der transgenen Linie Nr. 2013 (transformiert mit dem Konstrukt pPS41) bestimmt. Die Daten wurden anhand der konstitutiv exprimierten Kontrollgene UBC (Ubiquitin konjugierendes Enzym) und GAPDH (Glyceraldehyd-Phospat Dehydrogenase) normalisiert. Die im Gesamtblatt verbleibende Expression stammt aus der nichtabgezogenen oberen Blattepidermis und aus dem Phloem (Nebenaktivität des Promotors).
   C) durch Real-time reverse PCR-Analyse
      Es wurden Wildtyp-Pflanzen (Bobwhite) und die transgenen Linien Nr. 2013 und Nr. 2151 (transformiert mit dem pPS 41-Konstrukt) im Adultpflanzenstadium analysiert.
      Der Promotor wird vor allem in Blättern und Ähren stark exprimiert. In Stengel und Wurzeln wird das Transgen nicht oder nur schwach exprimiert.
13) Untersuchung der Mehltau-Resistenz von pPS41-transgenen Pflanzen
   Das Fahnenblatt adulter Pflanzen wurde abgeschnitten und in einem "detached leaf assay" mit Weizenmehltau inokuliert, zusammen mit Bobwhite Wildtyppflanzen. 7 Tage nach Inokulation wurde der Mehltaubefall bonitiert. Mittelwerte aus 3 unabhängigen Inokulationsexperimenten mit Pflanzen der T2 und T3 Generation. Sublinie 2088/2 exprimiert kein TAPERO und ist nicht erhöht resistent. Mittelwert "non-silenced" = Mittelwert aus allen Linien außer 2088/2 und allen Experimenten.
14) Sprosswachstum von pPS41-transgenen Pflanzen
   Pflanzen der T2 Generation wurden zusammen mit Bobwhite Wildtyppflanzen ausgesät und im Adultpflanzenstadium fotografiert.
15) Untersuchung der Mehltau-Resistenz von pWIR5-TaMlo-RNAi-transgenen Pflanzen
   Das Fahnenblatt adulter Pflanzen der T2 Generation wurde abgeschnitten und in einem "detached leaf assay" mit Weizenmehltau inokuliert, zusammen mit Bobwhite Wildtyppflanzen. 7 Tage nach Inokulation wurde der Mehltaubefall bonitiert. Je 2 Sublinien pro Linie wurden getestet.

### Beispiele:

In den nachfolgenden Beispielen wurden molekularbiologische Standardmethoden wie *E.coli* Transformation, Restriktionsverdau, Ligation, DNA Extraktion, PCR etc., wie sie im Stand des Technik bekannt sind, gemäss Sambrook et al. (2001), vide supra, durchgeführt. Für alle PCR-Reaktionen wurde "proofreading" *Pwo* Polymerase (Roche) verwendet.

### 1) Herstellung des Promotorkonstruktes aus GSTA1-Promotor und WIR1a-Intron (pPS18)

Die Herstellung erfolgte mehrstufig über folgende Vorläuferkonstrukte: pPS1, pPS3, pPS15. Alle Konstrukte enthielten das GUS Reportergen, um sie direkt im transienten Assay testen zu können.

### pPS1:

Ein 1.9 kb Promotorfragment des WIR1a Gens wurde mit *Pst*I aus einem rekombinanten pBluescript Klon herausgeschnitten und in die *Pst*I-Schnittstelle einer Expressionskassette vor das GUS-Gen kloniert. Die Expressionskassette basierte auf pBluescript und enthielt das GUS-Gen gefolgt vom Transkriptionsterminator des Weizen GSTA1-Gens. Da das GUS-Gen und der GSTA1-Transkriptionsterminator in den verwendeten finalen Konstrukten (siehe Beispiel 2) nicht mehr enthalten sind, wird auf eine detaillierte Beschreibung dieser Expressionskassette verzichtet. Das resultierende Konstrukt enthielt eine translationelle WIR1a::GUS Fusion.

### pPS3:

Mit den Adaptor-Primern 5' ATA TAT CTG CAG GGA GCC ACG GCC GTC CAC und 5' TAT CCC GGG CCC GTG CCT GGA CGG GAA wurde ein PCR Fragment von ca. 240 bp erzeugt und dessen Enden mit *Sma*I und *Pst*I geschnitten (auf Adaptor). Als PCR "template" diente der genomische WIR1a Klon. Das PCR Fragment enthielt die letzten 15 Aminosäuren des ersten Exons von WIR1a und das Intron inklusive "splice site" Akzeptor und wurde in pPS1, geschnitten mit *Pst*I (partiell) und *Sma*I und über Agarose-Gelelektrophorese gereinigt, ligiert. Das resultierende Konstrukt enthielt eine translationelle WIR1a::GUS Fusion mit dem WIR1 Intron vor dem GUS Gen. Zudem wurde eine Deletion von Aminosäuren Nr. 18-35 des ersten Exons von WIR1a eingeführt, um die Sekretion des WIR1a::GUS Fusionsproteins (durch Entfernen des Signalpeptids) zu verhindern.

### pPS15:

Der WIR1a Promotor wurde durch ein PCR-Fragment des GSTA1-Promotors ersetzt. Zu diesem Zweck wurde pPS3 mit *Xho*I und *Sna*BI (partiell) verdaut und die Vektorbande über Agarose-Gelelektrophorese gereinigt. Das GSTA1-Promotorfragment von ca. 2.3 kb Länge wurde mit den Adaptor-Primern 5'ATA TAT CTC GAG TCT AGA ACT AGT GGA TCC und 5'ATA TAT TAC GTA GTT TGT CCG TGA ACT TCA aus dem genomischen GSTA1 Klon mittels PCR amplifiziert und an den Enden mit *Xho*I und *Sna*BI geschnitten. Das PCR Fragment wurde mit der geleluierten pPS3 Bande ligiert, resultierend in einer translationellen Fusion des intronenthaltenden WIR1a Genfragmentes mit GUS, unter der Kontrolle des GSTA1 Promotors.

### pPS18:

pPS 15 wurde mit *Pst*I und *Sna*BI (partiell) verdaut, die Vektorbande über Agarose-Gelelektrophorese gereinigt und mit einem doppelsträngigen Oligonukleotid (5'GTA CAC AGG CAG CTA GCT CTC GAA ACC TCG CTC GAA ACG CA plus 5'CAT GTG TCC GTC GAT CGA GAG CTT TGG AGC GAG CTT TGC GT) ligiert. Dies ersetzte den Teil des WIR1a Gens um den Translationsstart (46 bp upstream bis 53 bp downstream des Translationsstarts) mit 42 bp der 5'UTR des WIR1a-Gens ohne das Translationsinitiationskodon ATG. Das resultierende Konstrukt enthielt eine transkriptionelle Fusion des intronenthaltenden WIR1a Genfragmentes mit GUS, unter der Kontrolle des GSTA1 Promotors.

### 2) Herstellung der verwendeten Konstrukte

### a) Expressionsvektor pPS24 (Oxalat-Oxidase-Expression unter der Kontrolle des erfindungsgemäßen Promotors)

Ein *Hind*III/*Sph*I Fragment von 745 bp Länge des Weizen gf-2.8 Gens (Oxalat-Oxidase; Acc. Nr. M63223) enthaltend den gesamten offenen Leserahmen (ORF) wurde in die pflanzliche Expressionskassette pGY1 subkloniert, was im Konstrukt pGermin (beschrieben in Schweizer et al., 1999) resultierte. Für diese Klonierung wurde das Oxalat-Oxidase-Fragment in einen Zwischenvektor ligiert, um das Fragment mittels der Restriktionsschnittstellen *Bam*HI und *Pst*I in pGY1 ligieren zu können. Aus pGermin wurde ein *Sma*I*lEco*RI Fragment von ca. 1 kb Länge, enthaltend das Oxalat-Oxidase-Gen und den CamV 35S Terminator, in den *Sma*I/*Eco*RI-geschnittenen und über Agarose-Gelelektrophorese gereinigten pPS18 Vektor ligiert. Das resultierende Konstrukt enthielt eine transkriptionelle Fusion des intronenthaltenden WIR1a Genfragmentes mit dem Oxalat-Oxidase-Gen unter der Kontrolle des GstA1 Promotors. Gegenüber pPS18 enthielt das Konstrukt nicht mehr den GstA1 Transkriptionsterminator, sondern denjenigen des CamV 35S Gens.

### b) Expressionsvektor pPS41 (TAPERO-Expression unter der Kontrolle des erfindungsgemäßen Promotors)

Aus pWIR3 (enthaltend eine transkriptionelle Fusion zwischen dem CamV 35S Promotor und TAPERO; Schweizer et al., 1999) wurde ein TAPERO-Fragment von ca. 1.2 kb Länge durch Restriktionsverdau mit *Sma*I und *Pst*I isoliert. Das TAPERO-Fragment wurde in Vektor pPS24, der mit *Sma*I und *Pst*I (partiell) verdaut und über Agarose-Gelelektrophorese gereinigt wurde, ligiert. Dies resultierte in einer transkriptionellen Fusion des intronenthaltenden WIR1a-Genfragmentes mit dem TAPERO-Gen (Acc. Nr. X56011), unter der Kontrolle des GstA1-Promotors, in dem das Oxalat-Oxidase Gen durch das TAPERO-Gen ausgetauscht wurde. Wie pPS24 enthält pPS41 den Transkriptionsterminator des CamV 35S Gens.

### c) Expressionsvektor pWIR5-TaMlo-RNAi (Expression des Mlo-RNAi-Konstrukts unter der Kontrolle des erfindungsgemäßen Promotors)

Zunächst wurde das im Vektor pGEM-Teasy subklonierte 3. Intron des *Mla1* Resistenzgens aus Gerste (ca. 1.1 kb) mittels *Eco*RI und *Pst*I isoliert und in den ebenfalls *Eco*RI und *Pst*I geschmittenen Vektor pBSw41 (pBluescript-Derivat mit partieller *TaMlo1* cDNA, kloniert von Candace Elliott im Rahmen ihrer Dissertation; GenBank accession no. AF361933) ligiert. Aus diesem Konstrukt wurde das *Mla1* Intron zusammen mit einem Teil der codierenden Sequenz des *TaMlo1*-Gens als ca. 1.55 kb *Pst*I/*Msc*I-Fragment isoliert (= Fragment 1). Parallel hierzu wurde per PCR aus dem Plasmid pBSw41 mit den Oligonukleotiden T3 (Standard-Sequenzier-Primer für pBluescript) und TaMlol-1 (5' GTC GCA TGC CTG TCC ACA CGA AAT GTG C 3', *Sph*I Restriktionsschnittstelle unterstrichen) ein ca. 450 bp großes Fragment amplifiziert. Nachfolgend wurde das PCR-Fragment mit den Restriktionsenzymen *Pst*I und *Sph*I verdaut (= Fragment 2). Der Vektor pPS24 (Promotor + Oxalat-Oxidase, siehe oben) wurde mittels Restriktionsverdau mit *Sma*I und *Sph*I geöffnet und das herausgeschnittene Oxalat-Oxidase-Genfragment verworfen. In einer Drei-Komponenten-Ligation wurden sodann die oben beschriebenen Fragmente 1 und 2 in den *Sma*I*lSph*I geschnittenen Vektor pPS24 ligiert. Bei dieser Ligation sind die Enden der *Msc*I und *Sma*I geschnittenen Komponenten kompatibel, da es sich bei beiden um sogenannte "stumpfe Enden" (blunt ends) handelt. Das resultierende Konstrukt (pTaMlol RNAi) enthält ca. 300 bp des *TaMlo1-*Gens sowie ca. 150 bp Polylinker/Adaptor-Sequenz als "inverted repeats", separiert durch das *Mla1*-Intron. Die Kontrolle dieser Transkriptionseinheit unterliegt dem GstA1 Promotor.
Anmerkung: Das hier aus historischen Gründen als *TaMlo1* bezeichnte Gen erhielt später die Bezeichnung *TaMloA1* (Elliott et al., 2002). Mol. Plant Microbe Interact. 15: 1069-1077 (2002).

### 3) Transformation der Weizenpflanzen

Weizenpflanzen (cv. Bobwhite) wurden in Phytokammern für 40 Tage bei 15°C tagsüber und 12°C nachts unter Kurztagbedingungen (10h/d, ca. 600 µE) und nachfolgend im Gewächshaus bei 18/16°C und einer Photoperiode von mindestens 16 h angezogen. Die Ähren wurden entweder unmittelbar verwendet oder bis zu 5 Tage bei 4°C aufbewahrt. Die von der Ähre abgenommenen Karyopsen wurden für 2 Minuten mit 70% Ethanol und dann für 15 bis 20 Minuten in 5% Natriumhypochlorit-Lösung/ 0,1% Tween 20 oberflächensterilisiert und schließlich viermal mit sterilem Aqua bidest gewaschen.

Unreife Embryonen mit einer Größe von 0,5 bis 1,5 mm wurden unter sterilen Bedinungen aus den Karyopsen herauspräpariert und mit dem Scutellum nach oben auf Kallusinduktions-Medium in Petrischalen aufgelegt (Basismedium nach Murashige Skoog (1962) mit 2 mg/l 2,4-D, 40 g Maltose-Monohydrat, 500 mg/l L-Glutamin, 100 mg/l Caseinhydrolysat, 5 µM CuSO₄ und 0,25% Phytagel). Die Kulturen wurden bei 25°C im Dunkeln inkubiert.

Fünf bis sieben Tage nach Isolierung der Embryonen erfolgte die biolistische Transformation. Vier bis sechs Stunden vor dem Partikelbeschuss wurden die bereits proliferierenden Embryonen auf neues Medium mit verringertem Wasserpotenzial übertragen (wie oben, supplementiert mit 0,3 M Mannitol) und bei 25°C im Dunkeln inkubiert.

Das Plasmid pAHC20 (Christensen and Quail 1996), das das Phosphinothricinacetyltransferase-codierende *bar*-Gen enthält, wurde in einem molaren Verhältnis von 1:1 mit einem zu co-transformierenden Vektor gemischt. Insgesamt wurden dann 10 µl Plasmid-DNA-Lösung auf die Partikel von 25 µl einer 60 mg/l Goldsuspension präzipitiert. Für einen Beschuss wurden 30 µg Partikel in 5 µl Ethanol auf einen Makrocarrier aufgetragen. Der Beschuss erfolgte entsprechend den Herstellerangaben der DuPont PDS-1000/He.

Zwölf bis 16 Stunden nach dem Partikelbeschuss wurden die Explantate auf neues Kallusinduktions-Medium (wie für die Vorkultur der Embryonen) übertragen und 10 Tage bei 25°C im dunkeln inkubiert.

Die Kalli wurden danach auf Differenzierungsmedium übertragen (Basismedium nach Murashige and Skoog (1962) mit 20 g/l Saccharose, 5 µM CuSO₄, 0,25% Phytagel und 3 mg/l Bialaphos) und bei einer Photoperiode von 16h bei 200 µE und 25°C inkubiert.

Nach 2 Wochen erfolgte die Übertragung der nicht verbräunten Kalli auf Regenerationsmedium (Basismedium nach Murashige and Skoog (1962) mit 20 g/l Saccharose, 0,25% Phytagel und 4 mg/l Bialaphos) und eine weitere Inkubation bei einer Photoperiode von 16h bei 200 µE und 25°C.

Nach weiteren 2 Wochen wurden die entstandenen Sprosse vereinzelt, in Kulturröhrchen mit Regenerationsmedium übertragen und bei einer Photoperiode von 16h bei 200 µE und 25°C weiterkultiviert.

Die Identifizierung transgener Regenerate erfolgte per PAT-Aktivitätstest von Blattextrakten nach Spencer et al. (1990) bzw. durch Amplifizierung Transgenspezifischer Sequenzen aus genomischer DNA der Kandidatenpflänzchen und/oder Southem Blot unter Verwendung einer entsprechenden Sonde.

Die Transformationseffizienz der Methode lag in Abhängigkeit von der Qualität des Ausgangsmaterials zwischen 0,5 bis 3 transgenen Pflanzen pro 100 kultivierten Embryonen.

### 4) In situ Oxalat-Oxidase-Aktivität in Pflanzen mit dem pPS24-Konstrukt

Blattsegmente von Bobwhite Wildtyppflanzen oder von pPS24-transgenen Weizenpflanzen der T3 Generation wurden unter Vakuum mit Oxalat-Oxidase-Nachweislösung (2.5 mM Oxalsäure, 3.5 mM freies EDTA, 0.6 mg/ml 4-Chlor-1-Naphthol, 50 µg/ml Peroxidase aus Meeretich, 20% v/v Ethanol, mit Tris Base auf pH 4.0 eingestellt) infiltriert und über Nacht bei +37°C inkubiert. Nach Entfernen der Nachweislösung wurden die Blätter für weitere 24 h bei +4°C in H₂O inkubiert. Danach wurden die Blätter von Hand mittels Skalpell in dünne Segmente quergeschnitten und mikroskopiert. Die Phasenkontrast-Lichtmikroskopie erfolgte mit einem Zeiss Axiophot bei 100-facher Vergrösserung. Zellen mit Oxalat Oxidase Expression besitzen violett gefärbte Zellwände.

### 5) Nachweis des TAPERO-Transgens in pPS41-transgenen Pflanzen durch Northern-Blot-Analyse

Blätter von Bobwhite und von pPS41-transgenen Pflanzen der T2 Generation (je ca. 1 g Frischgewicht, FG), beide im Fahnenblattstadium, wurden in flüssigen Stickstoff homogenisiert, bis feines Pulver entstand. Das Pulver wurde zu 3 ml RNA-Extraktionspuffer (0.5 M Tris-Cl pH 8.0; 0.25 M Na-EDTA; 5% (w/v) SDS) und 1,5 ml puffergesättigtem Phenol gegeben (15 ml Plastik-Röhrchen) und gut geschüttelt. Die Extrakte wurden für 30 min bei 4000 rpm-5000 rpm, 20°C zentifugiert (swing out, Heraeus Varifuge). Es wurde 1,5 ml Chloroform zugegeben (ohne Überstand abzugießen) und das Röhrchen mehrere Male invertiert. Die Extrakte wurden für 30 min bei 4000 rpm-5000 rpm, 20°C rezentifugiert und der Überstand vorsichtig in ein neues Röhrchen (15 ml Plastik-Röhrchen) gegossen. Die RNA wurde durch Zugabe von 3 ml 6 M LiCl gefällt (über Nacht, 4°C). Die gefällte RNA wurde für 30 min bei 12500 rpm, 4°C zentifugiert (Festrotor, Hermle Z360K), die RNA-Pellets wurden in 500-1000 µl 70% Ethanol aufgenommen (RNA löst sich nicht) und in Eppendorf röhrchen überführt. Die Proben wurden für 10 min bei 14000 rpm, 4°C zentrifugiert (Festrotor, Eppendorf Centrifuge 5417R) und der Überstand abgehoben. Die RNA-Pellets wurden 5 min bei 37°C getrocknet, in 100 µl-200 µl TE aufgenommen und 5-10 min bei 75°C gelöst. Die denaturierende Agarose-Gelelektrophorese der RNA in formaldehydhaltigen Gelen und der Transfer auf Nylonmembranen (Hybond N, Amersham) erfolgte gemäss Standardprotokollen (Sambrook et al., vide supra). Pro Probe wurden 10 µg RNA aufgetragen.
Die radioaktive Sondenmarkierung mit α ³²P-dCTP erfolgte nach der Methode des "random prime labelling" unter Verwendung eines Kits (Roche). Die Hybridisierung erfolgte über Nacht bei 65°C in CHURCH Puffer (0,5 M NaPhosphat pH 7,2; 1 % (w/v) BSA; 7 % (w/v) SDS; 1 mM Na₂ETDA). Die Blots wurden 2 x 15 min in Waschlösung (0.1 x SSC; 0.1 5 w/v) SDS) bei 65°C gewaschen und anschließend für 16-48 h gegen Phosporimager-Screens exponiert. Die exponierten Screens wurden mit einem Phosphorimagergerät (FujiFilm FLA 3000) eingescannt und als Bilddateien im TIFF Format exportiert.

### 6) Nachweis des TAPERO-Transgens in pPS41-transgenen Pflanzen durch Western-Blot-Analyse

Blattspitzen von Bobwhite und von pPS41-transgenen Pflanzen der T2 Generation, beide im Fahnenblattstadium, wurden in IWF Puffer (32 mM Na-Phosphat; 84 mM Citrat; pH 2.8; Spatelspitze Polyvinylpolypyrrolidon) homogenisiert. Die Homogenate wurden für 15 min bei 13.000 rpm und 4°C zentrifugiert. Die Überstände wurden mit 0.5 g/ml Ammoniumacetat versetzt und säurelösliche Proteine über Nacht bei 4°C gefällt. Die Proteine wurden für 30 min bei 13.000 rpm und 4°C zentrifugiert. Die Proteinpellets wurden in 50 µl/g FG Resuspensionspuffer (50 mM Tris-Cl pH 7.5; 20 % (v/v) Glycerin) aufgenommen. Zu 20 µl Probe wurden 5 µl 4-fach konzentrierter SDS Probenpuffer zugegeben, und die Proben wurden mit soviel (1-5 µl) gesättigter Tris-Lösung versetzt, bis der Farbumschlag des Bromphenolblaus zu Blau stattfand. Pro Spur wurden 12,5 µl gekochte Probe in denaturierender SDS-Polyacrylamidgelelektrophorese (15%iges Trenngel) nach einer Standardmethode unter Verwendung von Minigelapparaturen der Firma Bio-Rad aufgetrennt. Nach der Elektrophorese wurden die Gele entweder Coomassie gefärbt (als Beladungskontrolle) oder nach einer Standardmethode auf eine Nitrozellulosemembran übertragen (geblottet). Die Membranen wurden nach einer Standardmethode mit einem ersten, polyklonalen Antikörper(Verdünnung 1:2000), gerichtet gegen das Prx8 Protein aus Gerste (ein homologes Protein zu TAPERO), inkubiert, gefolgt vom zweiten Antikörper (Verdünnung 1:2000), der gegen Kaninchen-Antikörper gerichtet und an den alkalische Phosphatase gekoppelt war. Die TAPERO Proteinbanden wurden durch lokalisierte alkalische Phosphataseaktivität (BCIP/NBT Färbelösungen; Fertigtabletten (Roche)) nachgewiesen.

### 7) Nachweis der epidermisspezifischen Transgenexpression durch Northern-Blot-Analyse und Real-Time-PCR-Analyse

Die RNA-Extraktion und die Northern-Blot-Analyse wurden durchgeführt wie in Beispiel 5 beschrieben. Die Real-Time-PCR-Analyse erfolgte mit einem LightCycler®-Gerät (Roche, Mannheim, Deutschland) nach Herstellerangaben.

### 8) Mehltauresistenz in pPS41- bzw. pWIR5-TaMlo-RNAi-transgenen Pflanzen

Für den Resistenztest wurden adulte, im Gewächshaus angezogene pPS41- oder pWIR5-TaMlo-RNAi-transgene Weizenpflanzen mit voll entwickeltem, frisch geschobenem Fahnenblatt verwendet. Als Kontrollen dienten gleichzeitig angezogene Wildtyp-Pflanzen cv. Bobwhite. Die apikale Hälfte des Fahnenblattes wurde abgeschnitten und auf 0,5% (w/v) Phytoagar, der mit 20 ppm Benzimidazol versetzt war, in 20 x 20 cm großen Polycarbonat-Schalen aufgelegt. Pro Schale wurde eine transgene Sublinie (je 20 Blätter) plus Bobwhite Wildtyp (je 6 Blätter) aufgelegt. Die Blattsegmente wurden in einem Inokulationsturm mit Mehltausporen inokuliert, indem Sporen von 4 stark inokulierten Weizenblättern in den Turm eingeblasen wurden. Nach 5 min wurden die Schalen entfernt, geschlossen und bei 20°C und indirektem Tageslicht inkubiert. Sieben Tage nach Inokulation wurde der Mehltaubefall bonitiert unter Verwendung eines Klassenbonitursystems (Schweizer et al., 1995). Die Resistenz wurde bezogen auf die sich auf der jeweiligen Phytoagarplatte befindlichen Kontrollblätter berechnet.

### Literatur:

Christensen and Quail (1996) Transgenic Res. 5: 213-218.
Elliott et al., (2002). Molecular Plant Microbe Interactions 15: 1069-1077.
Murashige and Skoog (1962) Physiologia Plantarum 15: 473-497.
Schweizer, P., Vallélian-Bindschedler, L., and Mösinger, E. (1995). Heat-induced resistance in barley to the powdery mildew fungus Erysiphe graminis f.sp. hordei, Physiological and Molecular Plant Pathology 47, 51-66.
Schweizer, P., Pokorny, J., Abderhalden, O., and Dudler, R. (1999). A transient assay system for the functional assessment of defense-related genes in wheat, Mol Plant-Microbe Interact 12, 647-654.
Spencer et al. (1990) TAG 79: 625-631.

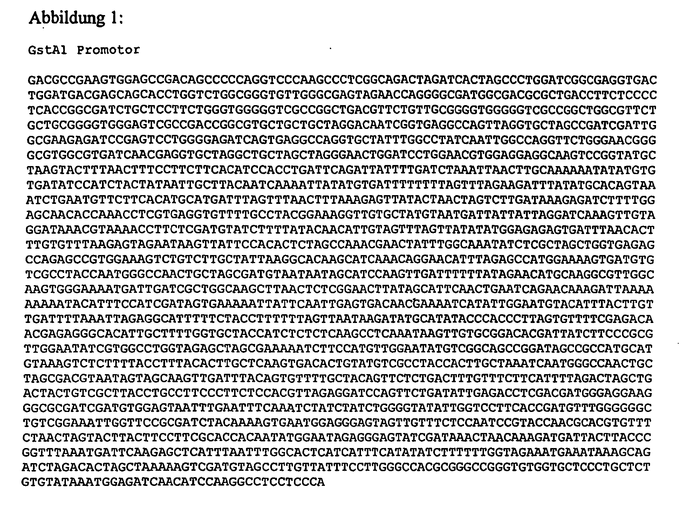

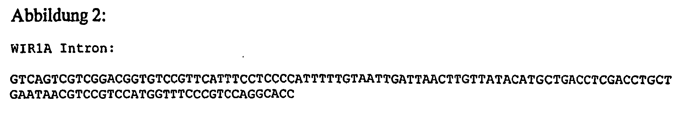

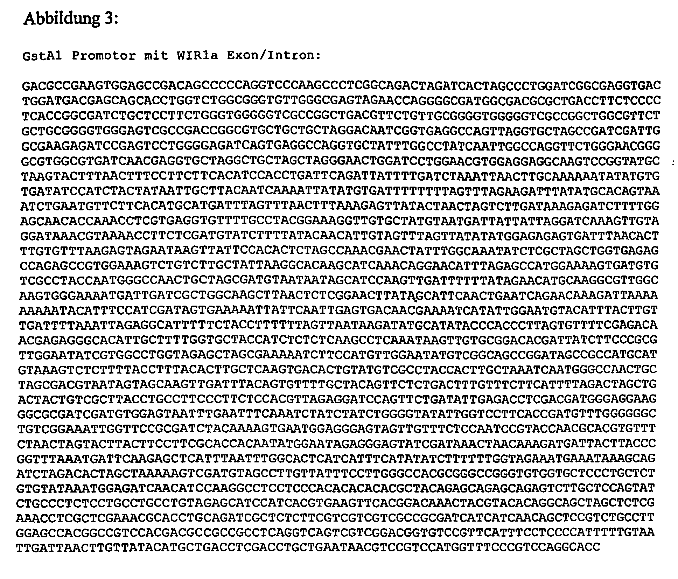

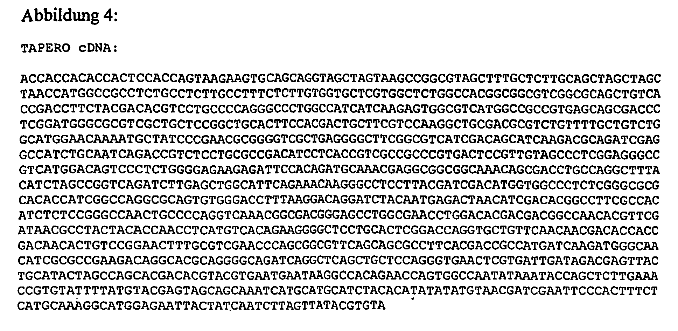

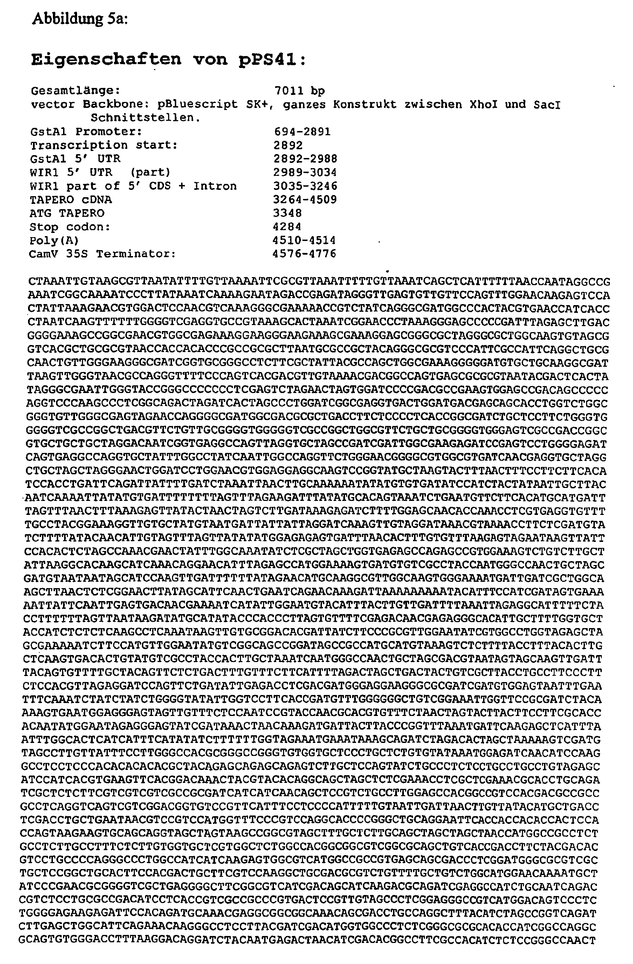

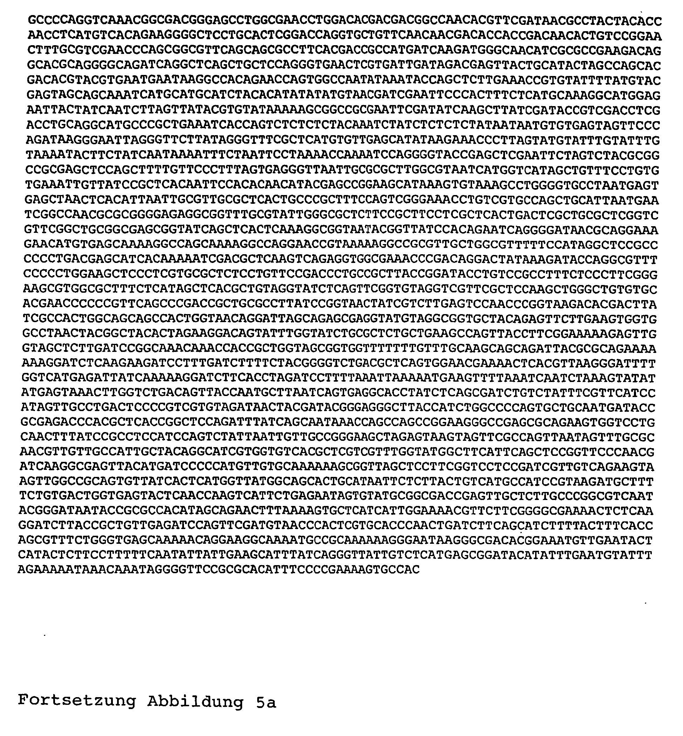

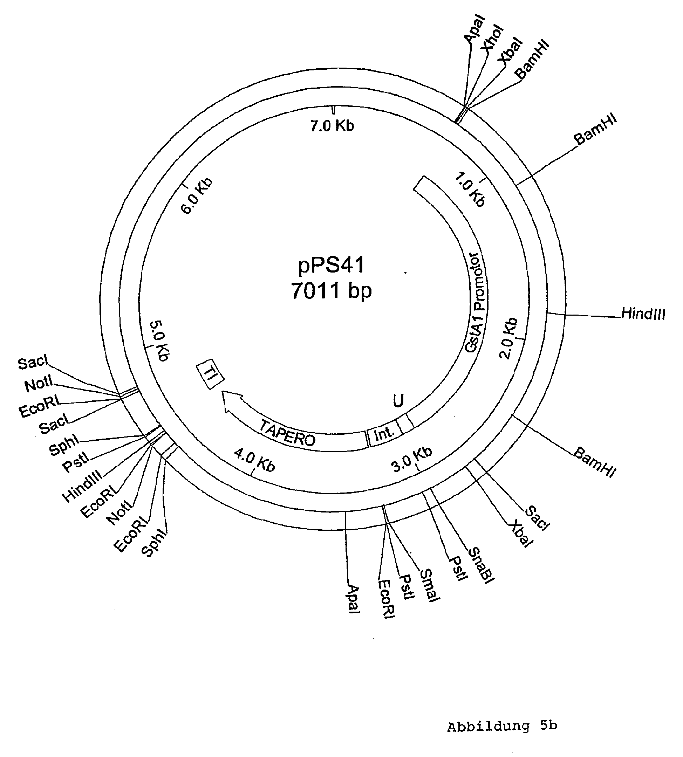

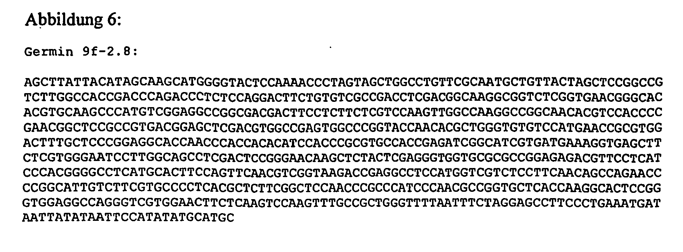

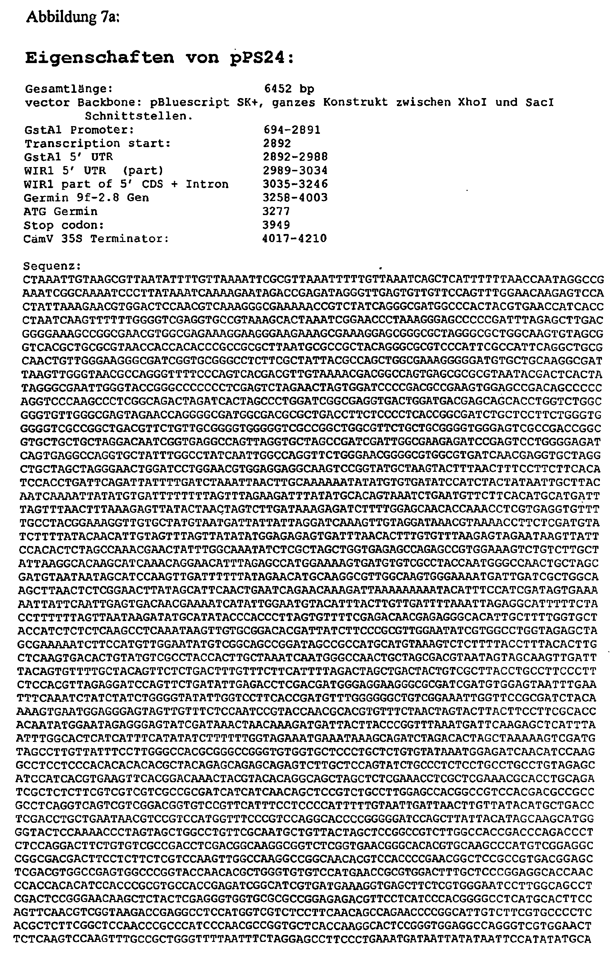

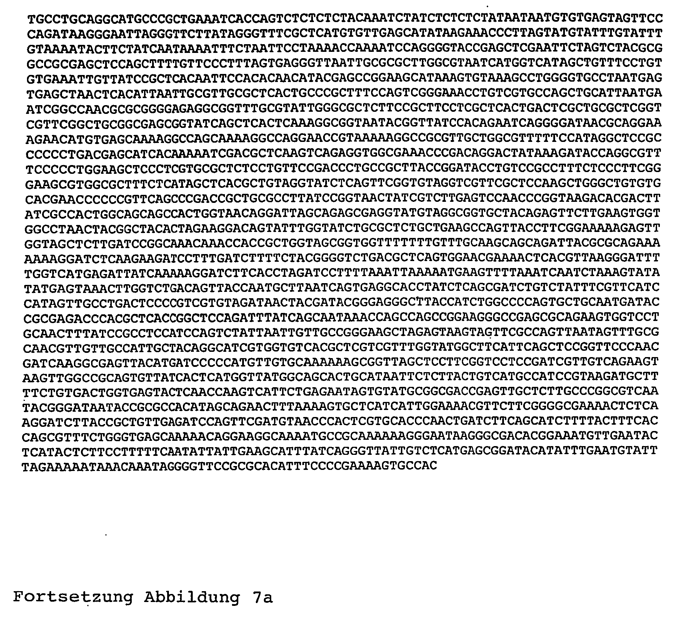

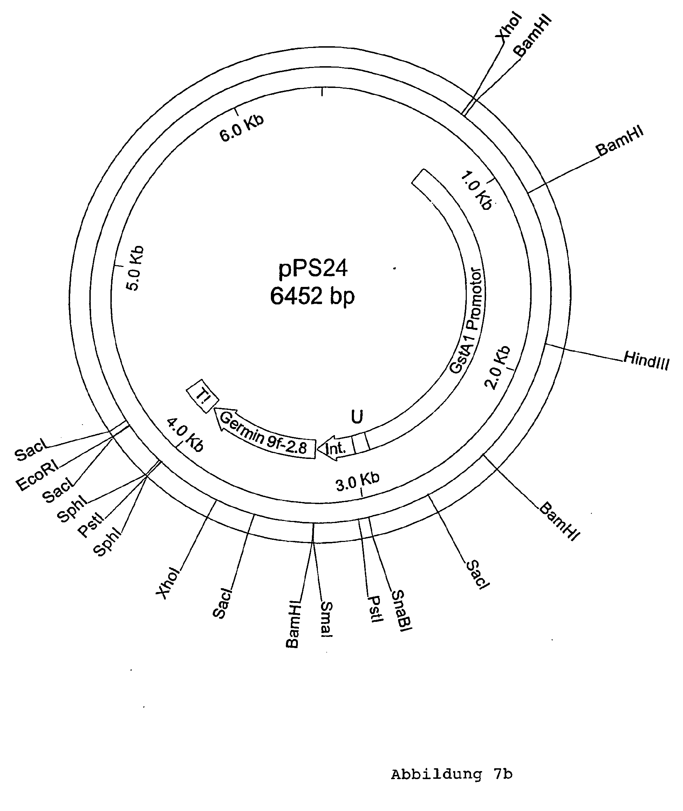

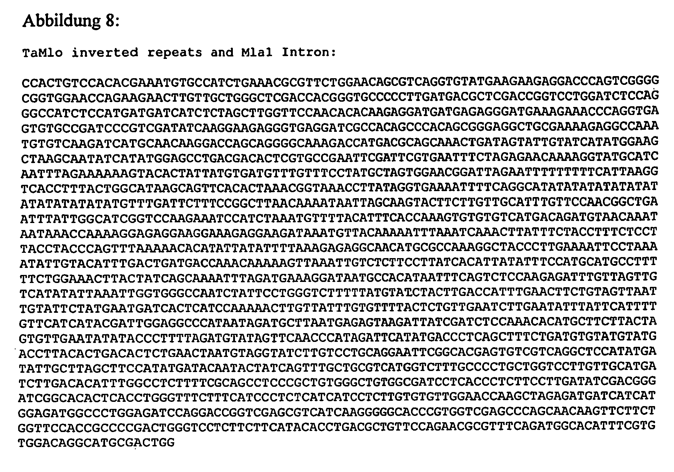

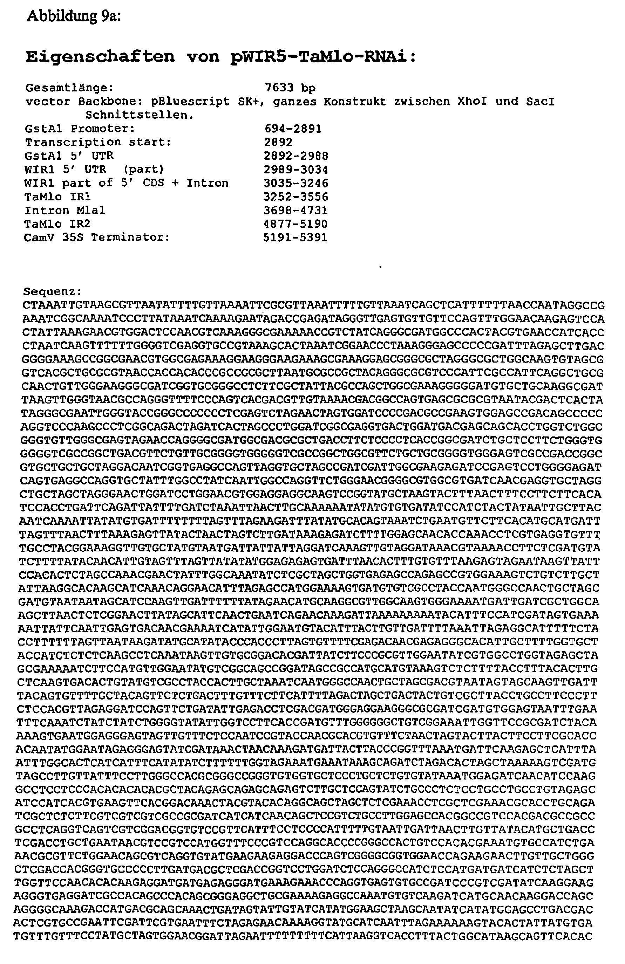

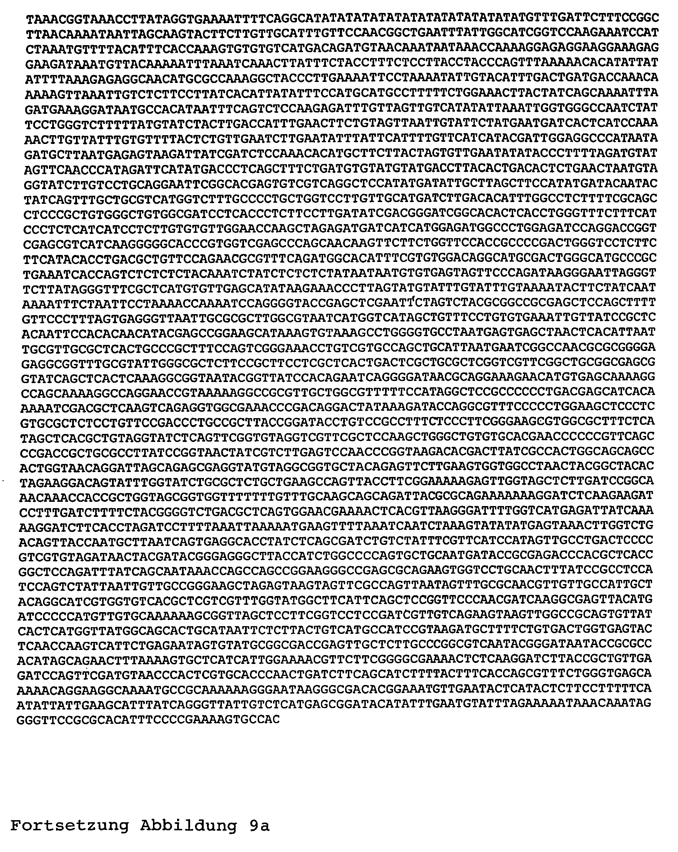

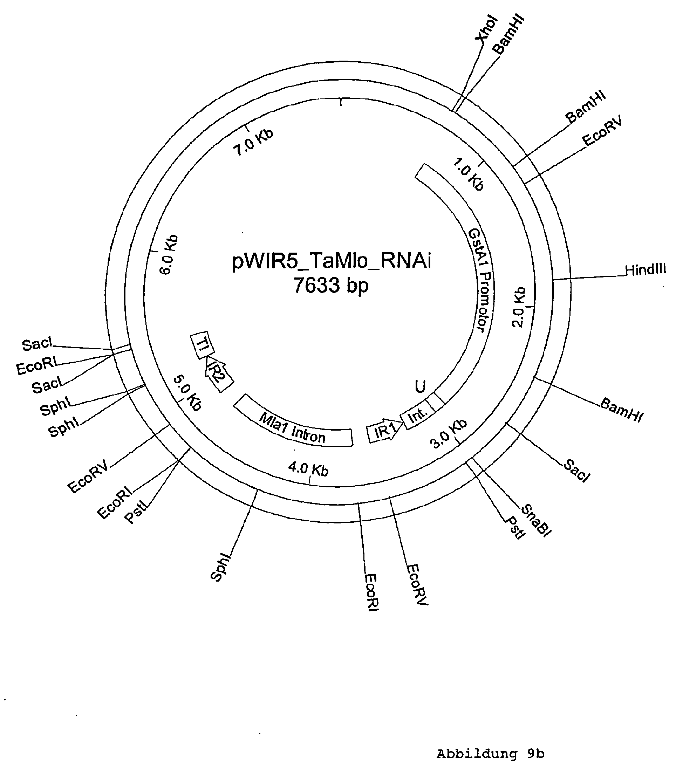

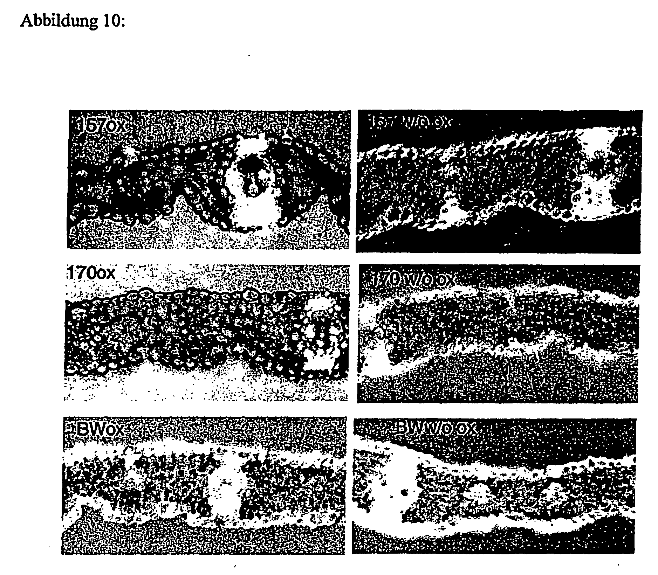

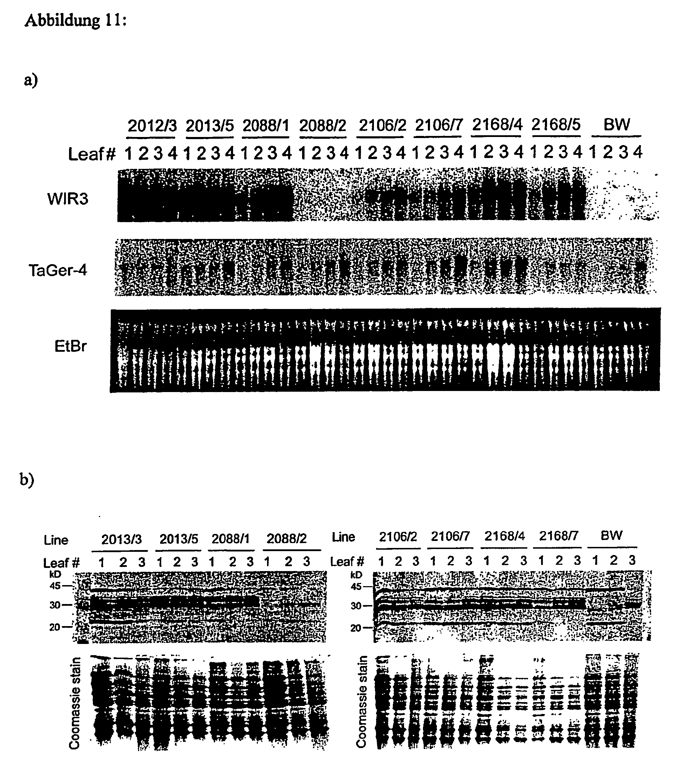

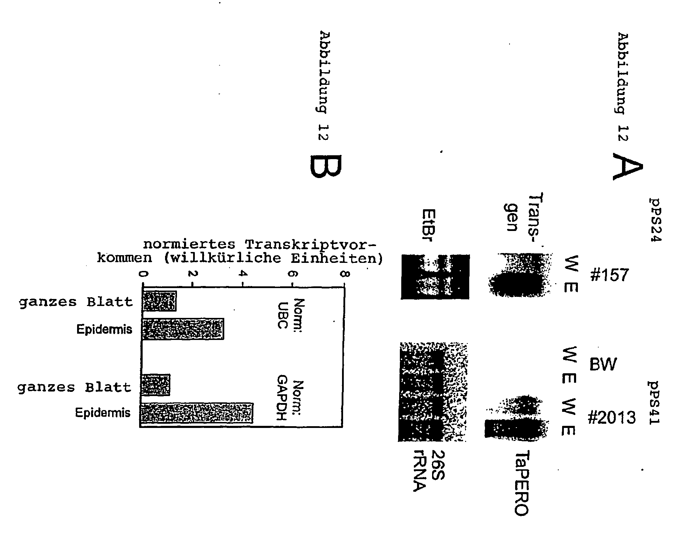

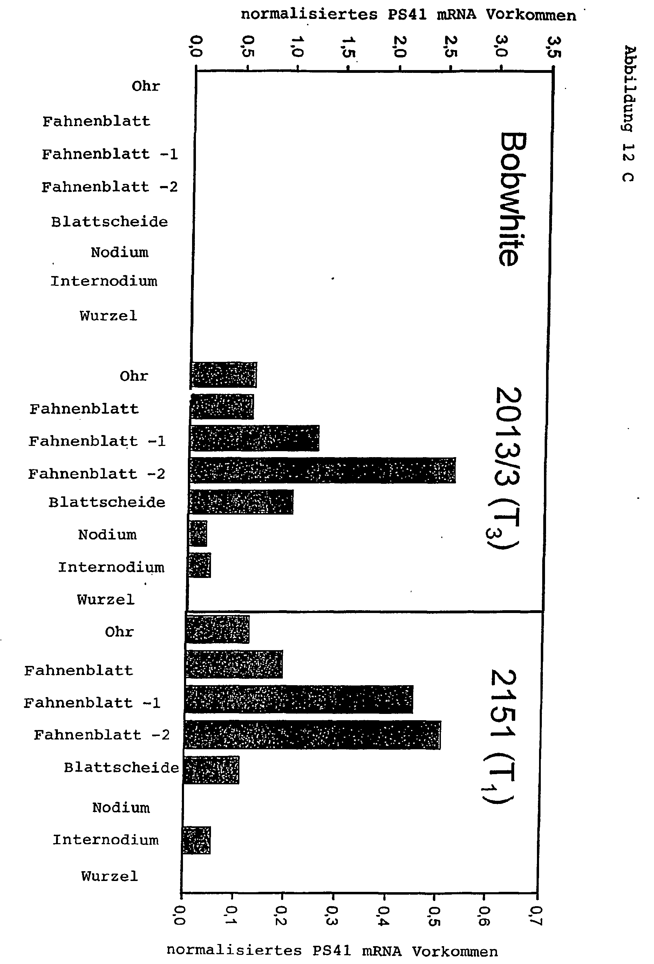

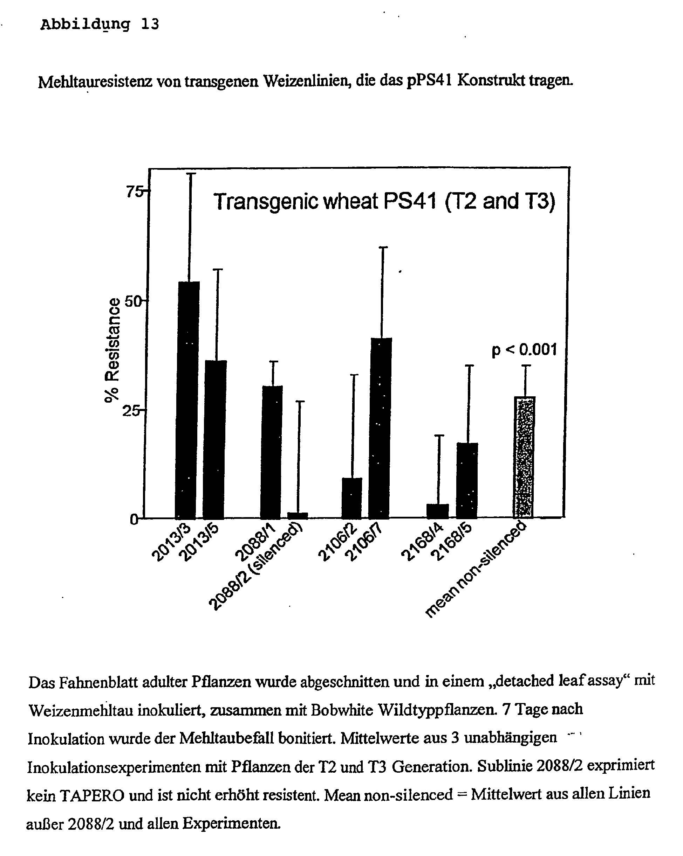

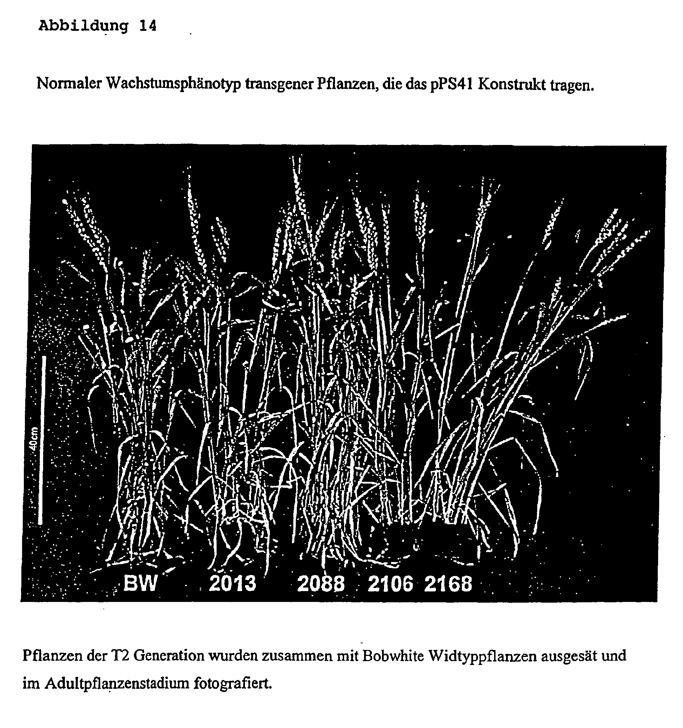

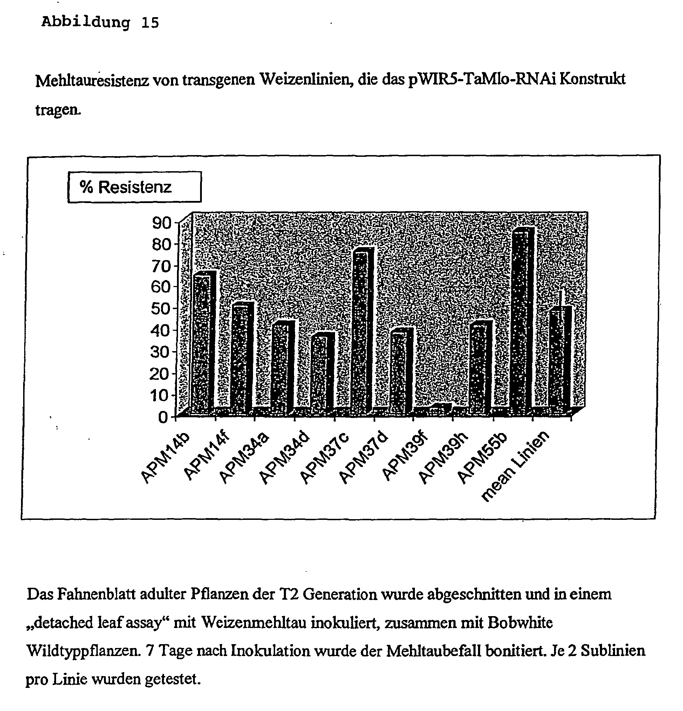

## Patentansprüche

1. Promotorregion mit Spezifität für die pflanzliche Epidermis, umfassend eine erste, aus dem Promotor des Gens GSTA1 stammende Sequenz umfassend eine TATA-Box und mindestens eines der Sequenzmotive ausgewählt aus der Gruppe bestehend aus
a) GTGGGGG,
b) ACGTGGA,
c) TCCACCT,
d) TATCCAT,
e) CATGCATG,
f) TGTAAAG,
g) CCTACCA,
h) AATAGTA;
und eine zweite, aus dem Intron des Gens WIR1a stammende Sequenz.

2. Promotorregion nach Anspruch 1,
**dadurch gekennzeichnet, dass** es sich bei der ersten Sequenz um SEQ ID Nr. 1 und bei der zweiten Sequenz um SEQ ID Nr. 2 handelt.

3. Promotorregion nach Anspruch 1 oder 2,
**dadurch gekennzeichnet, dass** sie ausgewählt ist aus der Gruppe bestehend aus
a) Promotorregionen, die die unter SEQ ID Nr. 3 angegebene Nukleinsäuresequenz umfassen,
b) Promotorregionen, die einen funktionalen Teil der unter SEQ ID Nr. 3 angegebenen Nukleinsäuresequenz umfassen, und
c) Promotorregionen, die eine Sequenz aufweisen, die unter stringenten Bedingungen mit der unter SEQ ID Nr. 3 angegebenen Nukleinsäuresequenz hybridisiert.

4. Chimäres Gen,
**dadurch gekennzeichnet, dass** es eine Promotorregion nach einem der Ansprüche 1 bis 3 in operativer Verknüpfung mit einer kodierenden Sequenz enthält.

5. Chimäres Gen nach Anspruch 4,
**dadurch gekennzeichnet, dass** seine Expression zu einem erhöhten Gehalt des von der kodierenden Sequenz kodierten Proteins in der Epidermis führt.

6. Chimäres Gen nach Anspruch 4 oder 5,
**dadurch gekennzeichnet, dass** die kodierende Sequenz aus einem Resistenzgen stammt.

7. Chimäres Gen oder rekombinantes Nukleinsäuremolekül nach Anspruch 5 oder 6,
**dadurch gekennzeichnet, dass** die kodierende Sequenz für eine Peroxidase oder eine Oxalat-Oxidase kodiert.

8. Chimäres Gen nach Anspruch 4,
**dadurch gekennzeichnet, dass** seine Expression die Expression des entsprechenden endogenen Gens in der Epidermis unterdrückt.

9. Chimäres Gen nach Anspruch 8,
**dadurch gekennzeichnet, dass** die kodierende Sequenz in antisense-Orientierung vorliegt.

10. Chimäres Gen nach Anspruch 8,
**dadurch gekennzeichnet, dass** die Unterdrückung der Expression des endogenen Gens durch RNA-Interferenz erfolgt.

11. Chimäres Gen nach einem der Ansprüche 8 bis 10,
**dadurch gekennzeichnet, dass** das endogene Gen, dessen Expression unterdrückt wird, das Mlo-Gen ist.

12. Rekombinantes Nukleinsäuremolekül, umfassend eine Promotorregion nach einem der Ansprüche 1 bis 3 oder ein chimäres Gen nach einem der Ansprüche 4 bis 11.

13. Rekombinantes Nukleinsäuremolekül nach Anspruch 12, zusätzlich umfassend transkriptionelle Terminationssequenzen.

14. Verfahren zur Herstellung transgener Pflanzen mit epidermisspezifischer Expression eines Transgens, umfassend die Schritte:
a) Herstellung eines rekombinanten Nukleinsäuremoleküls nach Anspruch 12 oder 13,
b) Übertragung des rekombinanten Nukleinsäuremoleküls aus a) auf pflanzliche Zellen und
c) Regeneration vollständig transformierter Pflanzen und, falls erwünscht, Vermehrung der Pflanzen

15. Transgene Pflanzen, enthaltend ein rekombinantes Nukleinsäuremolekül nach Anspruch 12 oder 13 oder hergestellt nach einem Verfahren nach Anspruch 14, sowie transgene Teile dieser Pflanzen und deren transgenes Vermehrungsmaterial, wie Protoplasten, Pflanzenzellen, Kalli, Samen, Knollen oder Stecklinge, sowie die transgenen Nachkommen dieser Pflanze.

16. Transgene Pflanzen nach Anspruch 15, bei denen es sich um monokotyledone Pflanzen handelt.

17. Transgene Pflanzen nach Anspruch 16, bei denen es sich um Poaceen handelt.

18. Transgene Pflanzen nach Anspruch 17, bei denen es sich um Weizen oder Gerste handelt.

19. Verwendung einer Promotorregion nach einem der Ansprüche 1 bis 3 zur epidermisspezifischen Expression von Transgenen in Pflanzen.

20. Verwendung nach Anspruch 19, wobei das Transgen ein Resistenzgen ist.

21. Verfahren zur Erhöhung der Pathogenresistenz in transgenen Pflanzen, umfassend die Schritte:
a) Herstellung eines rekombinanten Nukleinsäuremoleküls nach Anspruch 12 oder 13,
b) Übertragung des rekombinanten Nukleinsäuremoleküls aus a) auf pflanzliche Zellen und
c) Regeneration vollständig transformierter Pflanzen und, falls erwünscht, Vermehrung der Pflanzen.

22. Transgene Pflanzen mit erhöhter Pathogenresistenz, enthaltend ein rekombinantes Nukleinsäuremolekül nach einem der Ansprüche 12 bis 13 oder hergestellt nach einem Verfahren nach Anspruch 21, sowie transgene Teile dieser Pflanzen und deren transgenes Vermehrungsmaterial, wie Protoplasten, Pflanzenzellen, Kalli, Samen, Knollen oder Stecklinge, sowie die transgenen Nachkommen dieser Pflanze.

23. Transgene Pflanzen nach Anspruch 22, bei denen es sich um monokotyledone Pflanzen handelt.

24. Transgene Pflanzen nach Anspruch 23, bei denen es sich um Poaceen handelt.

25. Transgene Pflanzen nach Anspruch 24, bei denen es sich um Weizen oder Gerste handelt.

26. Transgene Pflanzen nach einem der Ansprüche 22 bis 25,
**dadurch gekennzeichnet, dass** sie eine erhöhte Resistenz gegen Echten Mehltau zeigen.

## Claims

1. Promoter region having specificity for the plant epidermis, comprising a first sequence originating from the promoter of the gene GSTA1, the first sequence comprising a TATA box and at least one of the sequence motifs selected from the group consisting of
a) GTGGGGG,
b) ACGTGGA,
c) TCCACCT,
d) TATCCAT,
e) CATGCATG,
f) TGTAAAG,
g) CCTACCA,
h) AATAGTA;
and a second sequence originating from the intron of the gene WIR1a.

2. Promoter region according to claim 1,
**characterized in that** the first sequence is SEQ ID No. 1 and the second sequence is SEQ ID No. 2.

3. Promoter region according to claim 1 or 2,
**characterized in that** it is selected from the group consisting of
a) promoter regions comprising the nucleic acid sequence given in SEQ ID No. 3,
b) promoter regions comprising a functional part of the nucleic acid sequence given in SEQ ID No. 3, and
c) promoter regions having a sequence, which hybridizes under stringent conditions with the nucleic acid sequence given in SEQ ID No. 3.

4. Chimeric gene,
**characterized in that** it contains a promoter region according to any of the claims 1 to 3 in operative linkage with a coding sequence.

5. Chimeric gene according to claim 4,
**characterized in that** its expression results in an increased content of the protein encoded by the coding sequence in the epidermis.

6. Cimeric gene according to claim 4 or 5,
**characterized in that** the coding sequence originates from a resistance gene.

7. Chimeric gene or recombinant nucleic acid molecule according to claim 5 or 6,
**characterized in that** the coding sequence encodes a peroxidase or an oxalate oxidase.

8. Chimeric gene according to claim 4,
**characterized in that** its expression suppresses the expression of the corresponding endogenous gene in the epidermis.

9. Chimeric gene according to claim 8,
**characterized in that** the coding sequence is in antisense orientation.

10. Chimeric gene according to claim 8,
**characterized in that** the suppression of the expression of the endogenous gene results from RNA-interference.

11. Chimeric gene according to any of the claims 8 to 10,
**characterized in that** the endogenous gene whose expression is suppressed is the Mlo-gene.

12. Recombinant nucleic acid molecule, comprising a promoter region according to any of the claims 1 to 3 or a chimeric gene according to any of the claims 4 to 11.

13. Recombinant nucleic acid molecule according to claim 12, further comprising transcription termination sequences.

14. Method for generating transgenic plants exhibiting epidermis specific expression of a transgene, comprising the steps:
a) generating a recombinant nucleic acid molecule according to claim 12 or 13,
b) transferring the recombinant nucleic acid molecule from a) to plant cells and
c) regenerating entirely transformed plants and, if desired, propagating the plants.

15. Transgenic plants, containing a recombinant nucleic acid molecule according to claim 12 or 13 or generated according to a method according to claim 14, as well as transgenic parts of said plants and their transgenic propagation material, like protoplasts, plant cells, calli, seeds, tubers or cuttings, as well as the transgenic offspring of said plant.

16. Transgenic plants according to claim 15, wherein said plants are monocotyledonous plants.

17. Transgenic plants according to claim 16, wherein said plants are poaceae.

18. Transgenic plants according to claim 17, wherein said plants are wheat or barley.

19. Use of a promoter region according to any of the claims 1 to 3 for epidermis specific expression of transgenes in plants.

20. Use according to claim 19, wherein the transgene is a resistance gene.

21. Method for increasing the pathogen resistance in transgenic plants, comprising the steps:
a) generating a recombinant nucleic acid molecule according to claim 12 or 13,
b) transferring the recombinant nucleic acid molecule from a) to plant cells and
c) regenerating entirely transformed plants and, if desired, propagating said plants.

22. Transgenic plants with increased pathogen resistance, containing a recombinant nucleic acid molecule according to one of the claims 12 to 13 or generated according to a method according to claim 21, as well as transgenic parts of said plants and their transgenic propagation material, like protoplasts, plant cells, calli, seeds, tubers or cuttings, as well as the transgenic offspring of said plant.

23. Transgenic plants according to claim 22, wherein said plants are monocotyledonous plants.

24. Transgenic plants according to claim 23, wherein said plants are poaceae.

25. Transgenic plants according to claim 24, wherein said plants are wheat or barley.

26. Transgenic plants according to any of the claims 22 to 25,
**characterized in that** they exhibit an increased resistance against mildew.

## Revendications

1. Région promotrice ayant une spécificité pour l'épiderme végétal, qui comprend une première séquence provenant du promoteur du gène GSTA1 comprenant une boîte TATA et au moins un des motifs de séquence choisis parmi le groupe constitué de
a) GTGGGGG,
b) ACGTGGA,
c) TCCACCT,
d) TATCCAT,
e) CATGCATG,
f) TGTAAAG,
g) CCTACCA,
h) AATAGTA ;
et une deuxième séquence provenant de l'intron du gène WIR1a.

2. Région promotrice selon la revendication 1,
**caractérisée en ce qu'**il s'agit pour la première séquence de SEQ ID n° 1 et pour la deuxième séquence de SEQ ID n° 2.

3. Région promotrice selon la revendication 1 ou 2,
**caractérisée en ce qu'**elle est choisie parmi le groupe constitué des
a) Régions promotrices qui comprennent la séquence d'acides nucléiques indiquée par SEQ ID n° 3,
b) Régions promotrices qui comprennent une partie fonctionnelle de la séquence d'acides nucléiques indiquée par SEQ ID n° 3, et
c) Régions promotrices ayant une séquence qui dans des conditions stringentes s'hybride avec la séquence d'acides nucléiques indiquée par SEQ ID n° 3.

4. Gène chimérique,
**caractérisé en ce qu'**il contient une région promotrice selon l'une quelconque des revendications 1 à 3 en association opérationnelle avec une séquence codante.

5. Gène chimérique selon la revendication 4,
**caractérisé en ce que** son expression conduit à une teneur accrue de la protéine codée par la séquence codante dans l'épiderme.

6. Gène chimérique selon la revendication 4 ou 5,
**caractérisé en ce que** la séquence codante provient d'un gène de résistance.

7. Gène chimérique ou molécule d'acide nucléique recombinante selon la revendication 5 ou 6,
**caractérisé en ce que** la séquence codante code une péroxydase ou une oxalate oxydase.

8. Gène chimérique selon la revendication 4,
**caractérisé en ce que** son expression réprime l'expression du gène endogène correspondant dans l'épiderme.

9. Gène chimérique selon la revendication 8,
**caractérisé en ce que** la séquence codante se trouve en orientation antisens.

10. Gène chimérique selon la revendication 8,
**caractérisé en ce que** la répression de l'expression du gène endogène s'effectue par ARN interférence.

11. Gène chimérique selon l'une quelconque des revendications 8 à 10,
**caractérisé en ce que** le gène endogène, dont l'expression est réprimée, est le gène Mlo.

12. Molécule d'acide nucléique recombinante comprenant une région promotrice selon l'une quelconque des revendications 1 à 3 ou un gène chimérique selon l'une quelconque des revendications 4 à 11.

13. Molécule d'acide nucléique recombinante selon la revendication 12 comprenant en outre des séquences de terminaison transcriptionnelle.

14. Procédé visant à la production de plantes transgéniques avec expression spécifique de l'épiderme d'un transgène, comprenant les étapes :
a) Production d'une molécule d'acide nucléique recombinante selon la revendication 12 ou 13,
b) Transfert de la molécule d'acide nucléique recombinante issue de a) dans des cellules végétales et
c) Régénération de plantes entièrement transformées et, si désiré, reproduction des plantes.

15. Plantes transgéniques contenant une molécule d'acide nucléique recombinante selon la revendication 12 ou 13 ou produites conformément à un procédé selon la revendication 14, ainsi que les parties transgéniques de ces plantes et leur matériel de reproduction transgénique comme les protoplastes, les cellules végétales, les cals, les graines, les tubercules ou les boutures, ainsi que les descendants transgéniques de ces plantes.

16. Plantes transgéniques selon la revendication 15 pour lesquelles il s'agit de plantes monocotylédones.

17. Plantes transgéniques selon la revendication 16 pour lesquelles il s'agit de Poacées.

18. Plantes transgéniques selon la revendication 17 pour lesquelles il s'agit de blé ou d'orge.

19. Utilisation d'une région promotrice selon l'une quelconque des revendications 1 à 3 favorisant l'expression spécifique de l'épiderme de transgènes dans les plantes.

20. Utilisation selon la revendication 19, dans laquelle le transgène est un gène de résistance.

21. Procédé favorisant l'augmentation de la résistance aux pathogènes dans les plantes transgéniques, comprenant les étapes :
a) Production d'une molécule d'acide nucléique recombinante selon la revendication 12 ou 13,
b) Transfert de la molécule d'acide nucléique recombinante issue de a) dans des cellules végétales et
c) Régénération de plantes entièrement transformées et, si désiré, reproduction des plantes.

22. Plantes transgéniques ayant une résistance aux pathogènes accrue, contenant une molécule d'acide nucléique recombinante selon l'une quelconque des revendications 12 à 13 ou produites conformément à un procédé selon la revendication 14, ainsi que les parties transgéniques de ces plantes et leur matériel de reproduction transgénique comme les protoplastes, les cellules végétales, les cals, les graines, les tubercules ou les boutures, ainsi que les descendants transgéniques de ces plantes.

23. Plantes transgéniques selon la revendication 22, pour lesquelles il s'agit de plantes monocotylédones.

24. Plantes transgéniques selon la revendication 23, pour lesquelles il s'agit de Poacées.

25. Plantes transgéniques selon la revendication 24, pour lesquelles il s'agit de blé ou d'orge.

26. Plantes transgéniques selon l'une quelconque des revendications 22 à 25, **caractérisé en ce qu'**elles expriment une résistance accrue contre l'oïdium.
